# EUROPÄISCHE PATENTSCHRIFT

(11) **EP 3 375 481 B1**
(45) Veröffentlichungstag und Bekanntmachung des Hinweises auf die Patenterteilung: **09.12.2020**
(21) Anmeldenummer: 18164024.4
(22) Anmeldetag: 25.09.2014
(51) Int. Cl.: A61M 39/26, F16K 15/18

(54) **VERBINDUNGSELEMENT**
CONNECTING ELEMENT
ÉLÉMENT DE LIAISON

(30) Priorität: 02.10.2013 DE 102013220073
(43) Veröffentlichungstag der Anmeldung: 19.09.2018
(62) Teilanmeldung aus: 14186337.3
(73) Patentinhaber: Parker Hannifin Manufacturing Germany GmbH & Co. KG, 33659 Bielefeld (DE)
(72) Erfinder: Aydin, Tolga, 74206 Bad Wimpfen (DE)
(74) Vertreter: Grauel, Andreas

(56) Entgegenhaltungen:
- WO-A1-2006/078355
- WO-A2-2009/111596
- WO-A2-2010/111546
- US-A- 5 901 942

## Beschreibung

### Technisches Gebiet

Die Erfindung betrifft ein Verbindungselement, insbesondere zum Verbinden von Gefäßen und zur Erzeugung einer Fluidverbindung zwischen an dem Verbindungselement angeschlossenen Gefäßen bzw. Elementen. Auch betrifft die Erfindung ein elastisches Federelement als Ventilelement.

### Stand der Technik

Insbesondere im medizinischen Anwendungsbereich ist es im Stand der Technik bekannt, dass flüssige Lösungen oder Medikamente mittels Spritzen aus einem Vorratsgefäß aufgenommen werden und Patienten verabreicht werden. Dazu ist bekannt, dass mit der Spritze die Flüssigkeit direkt über die Kanüle der Spritze aufgenommen wird und anschließend direkt dem Patienten verabreicht wird.

Auch ist es bekannt, dass auf das Vorratsgefäß ein Verbindungselement aufgesetzt wird, woran dann die Spritze angebracht werden kann, um die Flüssigkeit aus dem Vorratsgefäß auf die Spritze aufzuziehen. Zieht man die Spritze anschließend wieder davon ab, so kann dennoch Flüssigkeit aus der Spritze auslaufen, was insbesondere bei sehr teuren Medikamenten sehr nachteilig ist. Auch ist damit eine Verschmutzung möglich, weil austretende Medikamente oder Flüssigkeiten verloren werden können, was im medizinischen Bereich oder im Pflegebereich als nachteilig angesehen ist.

Auch ist es bekannt, dass insbesondere Infusionslösungen mittels Schlauchleitungen mit einer Kanüle verbunden werden können, wobei auch in solche Schlauchleitungen oder Kanülen Medikamente zudosiert werden können. Dies erfolgt dann über ein Verbindungselement, das den gleichen Nachteilen unterliegt, wie bereits oben beschrieben.

Die WO 2006/078355 A1 offenbart unterschiedliche Gestaltungen von Verbindungselementen, beispielsweise ein Verbindungselement mit einem zweiteiligen Gehäuse, wobei ein erster Aufnahmeraum mit einem ersten Anschlusselement und einem darin angeordneten ersten verlagerbaren Ventilelement in einem ersten Gehäuse vorgesehen ist und bei dem ein zweites Gehäuse mit einem zweiten Aufnahmeraum mit einem zweiten Anschlusselement mit einem darin angeordneten zweiten verlagerbaren Ventilelement vorgesehen ist, wobei das erste Gehäuse mit dem zweiten Gehäuse verschraubbar ist.

### Darstellung der Erfindung, Aufgabe, Lösung, Vorteile

Es ist die Aufgabe der Erfindung, ein Verbindungselement zu schaffen, welches eine sichere Verbindung erlaubt und dennoch bei einem Nichtverbinden einen sicheren und dichten Abschluss des damit verbundenen Gefäßes erlaubt. Auch ist die Aufgabe der Erfindung ein elastisches Federelement als Ventilelement zu schaffen, welches eine sichere Abdichtung bei gleichzeitiger einfacher Gestaltung erlaubt.

Dies wird erreicht mit den Merkmalen von Anspruch 1.

Die Erfindung betrifft ein Verbindungselement mit einem Gehäuse mit einem ersten Aufnahmeraum mit einem ersten Anschlusselement und einem darin angeordneten ersten verlagerbaren Ventilelement und mit einem zweiten Aufnahmeraum mit einem zweiten Anschlusselement mit einem darin angeordneten zweiten verlagerbaren Ventilelement, der erste und der zweite Aufnahmeraum sind miteinander fluidverbunden, wobei das erste Ventilelement elastisch verformbar ausgebildet und verlagerbar ist und das zweite Ventilelement elastisch verformbar ausgebildet und verlagerbar ist, wobei das erste Ventilelement zwischen zwei Positionen verlagerbar ist, wobei in der ersten Position eine Fluidverbindung zwischen dem ersten Aufnahmeraum und dem ersten Anschlusselement durch das erste Ventilelement unterbrochen ist und in der zweiten Position eine Fluidverbindung zwischen dem ersten Aufnahmeraum und dem ersten Anschlusselement durch das erste Ventilelement geöffnet ist und wobei das zweite Ventilelement zwischen zwei Positionen verlagerbar ist, wobei in der ersten Position eine Fluidverbindung zwischen dem zweiten Aufnahmeraum und dem zweiten Anschlusselement durch das zweite Ventilelement unterbrochen ist und in der zweiten Position eine Fluidverbindung zwischen dem zweiten Aufnahmeraum und dem zweiten Anschlusselement durch das zweite Ventilelement geöffnet ist, wobei das erste Ventilelement mit einem Kanal ausgebildet ist, welcher in der ersten Position des ersten Ventilelements verschlossen ist und in der zweiten Position des ersten Ventilelements geöffnet ist, wobei ein Hohldorn in dem ersten Aufnahmeraum angeordnet ist und in den Kanal eingreift, wobei das erste Ventilelement an dem zylindrischen Bereich abragende Federarme aufweist. Dadurch wird ein beidseitig abgedichtetes Verbindungselement geschaffen.

Gemäß der Erfindung ist das Gehäuse zumindest zweiteilig ausgebildet, wobei die zumindest zwei Gehäuseteile vorteilhaft miteinander verbunden sind, wie insbesondere nicht zerstörungsfrei lösbar miteinander verbunden sind. Dabei können die zumindest zwei Teile beispielsweise miteinander verschweißt oder verklebt sein. Wenn mehr als zwei Gehäuseteile miteinander verbunden werden, können diese miteinander verschweißt sein. Auch können nur manche von ihnen miteinander verschweißt sein. Andere können nur eingelegt oder verklebt sein.

Vorteilhaft ist es weiterhin, wenn der erste Aufnahmeraum und der zweite Aufnahmeraum von einem ersten und einem zweiten Gehäuseteil gebildet werden, wobei das erste Gehäuseteil den ersten Aufnahmeraum und den zweiten Aufnahmeraum bilden, wobei das zweite Gehäuseteil den ersten Aufnahmeraum deckelartig begrenzt. Das zweite Gehäuseteil ist dabei bevorzugt eine Art Deckel, welcher auf das andere erste Gehäuseteil aufgesetzt wird und einen Aufnahmeraum abschließt bzw. begrenzt.

Besonders vorteilhaft ist es, wenn der Hohldorn in das erste oder das zweite Gehäuseteil als drittes Gehäuseteil eingesetzt ist. So kann der Hohldorn mit einer Halteplatte in einen der Aufnahmeräume eingesetzt und gegebenenfalls mit dem Gehäuseteil verbunden sein.

Besonders vorteilhaft ist es, wenn das den Hohldorn bildende dritte Gehäuseteil mit dem ersten oder dem zweiten Gehäuseteil stoffschlüssig verbunden ist. Dabei kann das dritte Gehäuseteil mit einem der anderen Gehäuseteile verbunden sein.

Alternativ ist es auch vorteilhaft, wenn der Hohldorn mit dem ersten oder dem zweiten Gehäuseteil einteilig ausgebildet, wie insbesondere gespritzt ist. Dadurch lässt sich die Komplexität der Montage reduzieren.

Auch ist es vorteilhaft, wenn das erste Anschlusselement an dem ersten Gehäuseteil angeordnet oder ausgebildet ist und das zweite Anschlusselement an dem zweiten Gehäuseteil angeordnet oder ausgebildet ist. Dies bewirkt, dass die beiden Anschlusselemente sich gegenüber liegen können, wenn die jeweiligen Gehäuseteile entsprechend ausgebildet sind. Dadurch wird eine einfache Durchströmung erreicht.

Auch ist es vorteilhaft, wenn der erste Aufnahmeraum einen im Schnitt kreisförmigen Querschnitt aufweist und von einer umlaufenden Wand begrenzt ist, wobei der Aufnahmeraum von zwei etwa ebenen, insbesondere oberen und unteren, Wandbereichen begrenzt wird, wobei das erste Anschlusselement an einer der beiden Wände, insbesondere der oberen Wand, angeordnet oder aufgenommen ist und in der anderen Wand, insbesondere der unteren Wand, eine Fluidverbindung zu dem zweiten Aufnahmeraum vorgesehen ist.

Auch ist es vorteilhaft, wenn der erste Aufnahmeraum einen im Schnitt kreisförmigen Querschnitt mit nach außen ragenden Taschen aufweist und von einer im Radius veränderlichen umlaufenden Wand begrenzt ist, wobei der Aufnahmeraum von zwei etwa ebenen Wandbereichen, wie insbesondere einer oberen und unteren Wand, begrenzt wird, wobei das erste Anschlusselement an einer der beiden Wände, insbesondere der oberen Wand, angeordnet oder aufgenommen ist und in der anderen Wand, insbesondere der unteren Wand, eine Fluidverbindung zu dem zweiten Aufnahmeraum vorgesehen ist. Die Ausbildung der Taschen erlaubt die einfache Anordnung von Federarmen des Ventilelements. Dadurch ist die Lage der Federarme relativ gut definiert und auch unter Kraftbeaufschlagung ist eine sichere Anordnung der Federarme gewährleistet.

Besonders vorteilhaft ist es, wenn die Anzahl der Taschen größer oder gleich der Anzahl von Federarmen eines in den Aufnahmeraum eingesetzten Ventilelements ist.

Besonders vorteilhaft ist es, wenn in jeder Tasche ein Federarm des Ventilelements angeordnet ist. Dadurch ist die Lage der Federarme eindeutig definiert und auch bei der Montage können keine Fehler hinsichtlich eines Fehleinsetzens auftreten.

Auch ist es vorteilhaft, wenn das erste Anschlusselement ein hohlzylindrisches Element aufweist und das erste Ventilelement zumindest einen zylindrischen Bereich aufweist, welcher in dem hohlzylindrischen Element verlagerbar aufgenommen ist. Das zylindrische Element dient dabei dem Abdichten bzw. Freigeben einer Öffnung des Anschlusselements.

Weiterhin ist es gemäß der Erfindung, wenn das erste Ventilelement an dem zylindrischen Bereich abragende Federarme aufweist. Dadurch kann das Ventilelement entgegen der Rückstellkraft der Federarme verlagert werden.

Dabei ist es besonders vorteilhaft, wenn die Federarme im Wesentlichen den Kanten eines Tetraeders entsprechend von dem zylindrischen Bereich abragen. Dadurch wird eine sichere Positionierung der Federarme und damit des Ventilelements erreicht.

Auch ist es zweckmäßig, wenn die abragenden Federarme als gerade oder einfach oder mehrfach abgeknickte oder gekrümmte Federarme ausgebildet sind. Bei den gekrümmten oder abgeknickten Federarmen wird eine definierte Einfederung durch Biegen um eine Sollbiegestelle erreicht.

Besonders vorteilhaft ist es, wenn die Federarme des ersten Ventilelements in einem Winkel von 120° zueinander in der Ebene senkrecht zur Längsachse des zylindrischen Bereichs angeordnet sind.

Dabei ist es auch vorteilhaft, wenn die Federarme einen ersten Bereich aufweisen, welcher in einem Winkel von etwa 25 bis 30°, vorzugsweise 27°, zur Ebene senkrecht zur Längsachse des zylindrischen Bereichs angeordnet ist.

Auch ist es vorteilhaft, wenn die Federarme einen zweiten Bereich aufweisen, welcher in einem Winkel von etwa 35 bis 40°, vorzugsweise 37°, zur Ebene senkrecht zur Längsachse des zylindrischen Bereichs angeordnet ist.

Weiterhin ist es vorteilhaft, wenn die Federarme einen dritten Bereich aufweisen, welcher in einem Winkel von etwa 75 bis 80°, vorzugsweise 77°, zur Ebene senkrecht zur Längsachse des zylindrischen Bereichs angeordnet ist.

Durch die mehrfache Abknickung wird ein vorteilhaftes definiertes Biegeverhalten unter Last erreicht.

Besonders vorteilhaft ist es, wenn die Federarme bei Belastung auf den zylindrischen Bereich sich derart bogenförmig verformen, dass der zylindrische Bereich im Wesentlichen eine axiale Bewegung vollzieht.

Erfindungsgemäß ist es zweckmäßig, wenn der Kanal des Ventilelements auf einer Seite offen ist und auf der gegenüberliegenden Seite mit einem verschließbaren und öffenbaren Schlitz verschließbar ausgebildet ist. Dabei ist ein Schlitz beispielsweise ein Längsschlitz oder ein kreuz- oder sternförmiger Schlitz oder ein anderweitig ausgebildeter Schlitz.

Dabei ist es vorteilhaft, wenn der Hohldorn in den Kanal im unbelasteten Zustand des Ventilelements nicht oder nur teilweise eingreift und den Schlitz im geschlossenen Zustand nicht beaufschlagt, so dass der Kanal verschlossen ist.

Ebenso ist es vorteilhaft, wenn der Hohldorn in den Kanal im belasteten Zustand des Ventilelements nahezu vollständig ein- oder hindurchgreift und den Schlitz beaufschlagt oder durchstößt, so dass der Kanal geöffnet ist.

Erfindungsgemäß ist es zweckmäßig, wenn das zweite Anschlusselement ein hohlzylindrisches Element aufweist.

So ist es vorteilhaft, wenn das zweite Anschlusselement ein hohlzylindrisches Element aufweist und das zweite Ventilelement zumindest einen zylindrischen Bereich aufweist, welcher in dem hohlzylindrischen Element verlagerbar und/oder verformbar aufgenommen ist. Durch eine Kraftbeaufschlagung des zweiten Ventilelements kann dieses in einer Aufnahme des zweiten Anschlusselements verlagert und/oder verformt werden, um eine Fluidverbindung zu öffnen oder zu verschließen.

Dabei ist es vorteilhaft, wenn das zweite Ventilelement an dem zylindrischen Bereich abragende Federarme aufweist. Dadurch kann das zweite Ventilelement entgegen der Rückstellkraft der Federarme verlagerbar sein. Auch ist das zweite Federelement entgegen der Rückstellkraft aufgrund der eigenen Elastizität elastisch verformbar.
Dabei ist es vorteilhaft, wenn die Federarme im Wesentlichen den Kanten eines Tetraeders entsprechend von dem zylindrischen Bereich abragen.

Auch können die abragenden Federarme als gerade oder einfach oder mehrfach abgeknickte oder gekrümmte Federarme ausgebildet sein.

Besonders vorteilhaft ist es, wenn die Federarme des zweiten Ventilelements in einem Winkel von 120° zueinander in der Ebene senkrecht zur Längsachse des zylindrischen Bereichs angeordnet sind.

Weiterhin ist es vorteilhaft, wenn die Federarme einen ersten Bereich aufweisen, welcher in einem Winkel von etwa 35 bis 55°, insbesondere etwa 45°, zur Ebene senkrecht zur Längsachse des zylindrischen Bereichs geneigt angeordnet ist.

Auch ist es vorteilhaft, wenn die Federarme einen zweiten Bereich aufweisen, welcher in einer Ebene senkrecht zur Längsachse des zylindrischen Bereichs angeordnet ist.

Weiterhin ist es vorteilhaft, wenn die Federarme bei Belastung auf den zylindrischen Bereich sich derart bogenförmig verformen, dass der zylindrische Bereich im Wesentlichen eine axiale Bewegung vollzieht.

Auch ist es vorteilhaft, wenn sich der zylindrische Bereich des zweiten Ventilelements unter Belastung krümmt. Dadurch kann sich der zweite zylindrische Bereich von einer Dichtfläche des zweiten Aufnahmeraums abheben und eine Fluidverbindung frei geben.

Auch ist es zweckmäßig, wenn der zweite Aufnahmeraum endseitig eine nach radial innen gerichtete Einschnürung aufweist, woran sich das zweite Ventilelement im unbelasteten Zustand dichtend anlegt.

Vorteilhaft ist es, wenn ein elastisches Federelement mit einem zylindrischen Körper und davon abragenden Federarmen ausgebildet ist, wobei das Federelement aus einem elastischen Material gefertigt ist.

Dabei ist es vorteilhaft, wenn die abragenden Federarme als einfach oder mehrfach abgeknickte und/oder gekrümmte Federarme ausgebildet sind.

Auch ist es zweckmäßig, wenn die Federarme des ersten und/oder des zweiten Ventilelements in einem Winkel von 120° zueinander in der Ebene senkrecht zur Längsachse des zylindrischen Bereichs angeordnet sind.

Dabei ist es zweckmäßig, wenn die Federarme einen ersten Bereich aufweisen, welcher in einem Winkel von etwa 35 bis 55° zu einer Ebene senkrecht zur Längsachse des zylindrischen Bereichs angeordnet ist.

Auch ist es vorteilhaft, wenn die Federarme einen zweiten Bereich aufweisen, welcher in einer Ebene senkrecht zur Längsachse des zylindrischen Bereichs angeordnet ist.

Auch ist es vorteilhaft, wenn der Querschnitt eines Federarms rund oder oval oder mehreckig ausgebildet ist.

Auch ist es vorteilhaft, wenn der Querschnitt des Federarms oval ist, wobei der Querschnitt in einer Ebene durch die Mittelsenkrechte des Federelements einen kleineren Durchmesser aufweist als in der dazu senkrechten Richtung.

Auch ist es vorteilhaft, wenn der zylindrische Körper an einem Endbereich, welcher gegenüberliegend der Federarme angeordnet ist, eine umlaufende Dichtlippe oder einen umlaufenden Dichtbereich aufweist.

Auch ist es vorteilhaft, wenn die Dichtlippe oder der Dichtbereich als umlaufender Absatz ausgebildet ist.

Weitere vorteilhafte Ausgestaltungen sind durch die nachfolgende Figurenbeschreibung und durch die Unteransprüche beschrieben.

### Kurze Beschreibung der Zeichnungen

Nachstehend wird die Erfindung auf der Grundlage zumindest eines Ausführungsbeispiels anhand der Zeichnungen näher erläutert. Es zeigen:
- Fig. 1: einen Schnitt eines ersten Ausführungsbeispiels eines erfindungsgemäßen Verbindungselements,
- Fig. 2: eine perspektivische Ansicht eines Gehäuseteils,
- Fig. 3: eine perspektivische Ansicht eines Ventilelements,
- Fig. 4: eine perspektivische Ansicht eines Gehäuseteils,
- Fig. 5: eine perspektivische Ansicht eines Ventilelements,
- Fig. 6: eine perspektivische Ansicht eines Gehäuseteils,
- Fig. 7: eine Aufsicht auf das Verbindungselement von oben,
- Fig. 8: einen Schnitt eines Beispiels eines nicht erfindungsgemäßen Verbindungselements,
- Fig. 9: eine perspektivische Ansicht eines Gehäuseteils,
- Fig. 10: eine perspektivische Ansicht eines Ventilelements,
- Fig. 11: eine perspektivische Ansicht eines Gehäuseteils,
- Fig. 12: eine Aufsicht auf das Verbindungselement von oben,
- Fig. 13: eine perspektivische Ansicht eines Adapterelements,
- Fig. 14: eine perspektivische Ansicht einer Öffnung des Hohldorns,
- Fig. 15: eine perspektivische Ansicht einer Öffnung des Hohldorns,
- Fig. 16: eine perspektivische Ansicht einer Öffnung des Hohldorns, und
- Fig. 17: einen Schnitt eines weiteren Ausführungsbeispiels eines erfindungsgemäßen Verbindungselements.

### Bevorzugte Ausführung der Erfindung

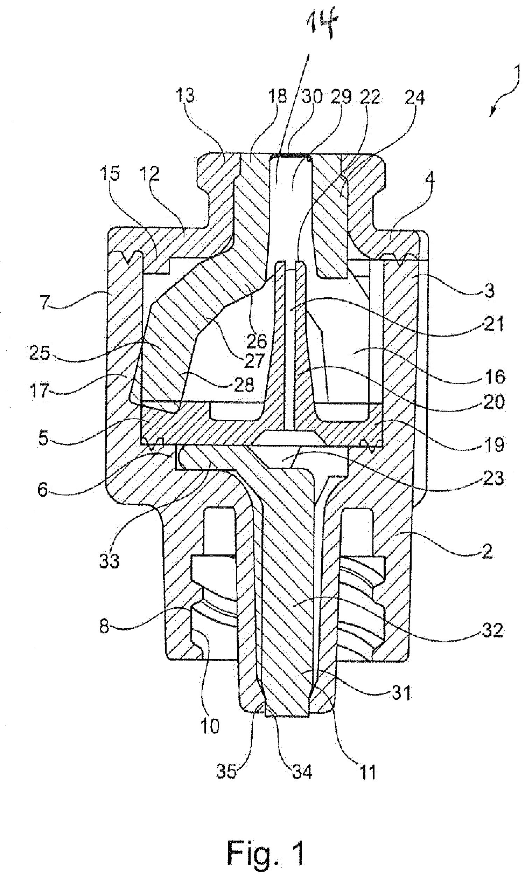

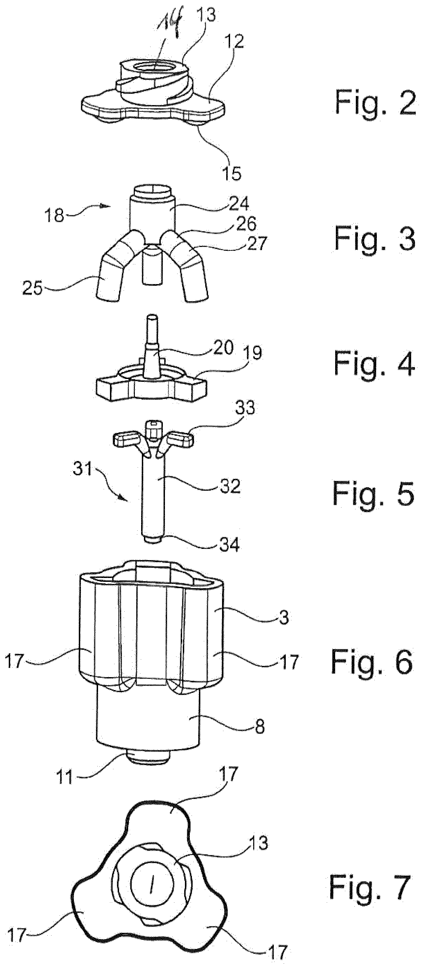

Die Figuren 1 bis 7 zeigen ein erstes Ausführungsbeispiel eines erfindungsgemäßen Verbindungselements 1 in verschiedenen Darstellungen. Die Figur 1 zeigt das Verbindungselement 1 im Schnitt, die Figuren 2 bis 6 zeigen eine Explosionsdarstellung in perspektivischer Ansicht, die Figur 7 zeigt eine Aufsicht.

Das Verbindungselement 1 ist mit einem Gehäuse 2 ausgebildet. Das Gehäuse 2 besteht aus zumindest zwei Gehäuseteilen, wobei in diesem Ausführungsbeispiel drei Gehäuseteile 3, 4, 5 vorgesehen sind. Die Gehäuseteile 3, 4, sind miteinander verbunden, wie miteinander verschweißt ausgebildet. Das Gehäuseteil 5 ist in das Gehäuseteil 3 eingesetzt und auch dort mit dem Gehäuseteil 3 verbunden.

Das erste Gehäuseteil 3 weist einen Boden 6 mit einer daran im Winkel von 90° abragenden umlaufenden Wand 7 auf. Die Wand 7 ist dabei im Wesentlichen axial ausgerichtet.

Der Boden 5 ist mit dem Anschlusselement 8 versehen. Dazu ist das Anschlusselement 8 als Anschlussstutzen an dem Boden 6 angebracht. Vorteilhaft ist es mit dem Boden 6 einteilig verbunden oder ausgebildet. Dabei kann mit dem Anschlusselement 8 ein zu verbindendes Element verbunden, wie verschraubt, werden. Dazu ist ein Gewinde 10 vorgesehen, mittels welchem das zu verbindende Element verschraubt werden kann.

Das Gehäuseteil 3 weist einen rohrförmigen Bereich 11 auf, welcher in axialer Richtung von dem Boden 6 abragt und in entgegengesetzter Richtung der Wand 7 vom Boden 6 abragt.

Das zweite Gehäuseteil 4 ist deckelartig mit einem Boden 12 ausgebildet, von dem sich in axialer Richtung nach oben ein Anschlussstutzen 13 mit einer Fluidöffnung 14 erstreckt. In die entgegengesetzte Richtung ist ein Vorsprung 15 vorgesehen, der in die Wand 7 radial innen und in axialer Richtung eingreift. Der Vorsprung 15 ist, wie in Figur 2 dargestellt, segmentartig ausgebildet und über den Umfang des Bodens 12 verteilt.

Wie in der Figur 1 zu erkennen ist, sind die beiden Gehäuseteile 3, 4 an der Stirnseite der Wand 7 miteinander verbunden, wie verschweißt. Auch kann alternativ ein Verkleben, Verpressen, Verclipsen etc. anwendbar sein.

Das Gehäuse 2 bildet einen ersten Aufnahmeraum 16, wobei dieser Aufnahmeraum innerhalb des Gehäuseteils 3 im vom Gehäuseteil 4 abgeschlossenen Bereich angeordnet ist. Dabei wird der Aufnahmeraum 16 durch die Wand 7 umgeben.

Die Wand 7 ist gemäß der Figuren 1 und 6 nicht vollständig rund ausgebildet, sondern weist nach radial außen vorstehende Taschen 17 auf, die zur Aufnahme von Federarmen des Ventilelements dienen. Dabei sind die Taschen 17 als nach radial außen abstehende Bereiche eines Kreises ausgebildet, wobei der Radius der Wand 7 zwischen den Taschen 17 geringer ist.

Im Aufnahmeraum 16 ist das dritte Gehäuseteil 5 angeordnet. Dieses weist eine Bodenplatte 19 und einen davon abragenden Hohldorn 20 auf, welcher einen durchgehenden Kanal 21 aufweist. Am oberen Ende des Hohldorns weist der Dorn zwei gegenüberliegende Vorsprünge 22 auf, die den Kanal zwischen sich in lateraler Richtung verlaufend enden lassen. Der Kanal 21 ist hin zum zweiten Aufnahmeraum 23 offen.

Im Aufnahmeraum 16 ist weiterhin ein Ventilelement 18 angeordnet.

Nach oben am Boden 12 des Gehäuseteils 4 ist das Anschlusselement 13 vorgesehen, wie bereits oben beschrieben. Wird ein mit dem Anschlusselement 13 zu verbindendes Element, wie beispielsweise eine Spritze oder ähnliches, bzw. ein daran angebrachter Stutzen in das Anschlusselement 13 eingesteckt oder eingeschraubt, so soll eine Fluidkommunikation zwischen dem zu verbindenden Element und dem Aufnahmeraum 16 erfolgen können. Wird kein zu verbindendes Element in das Anschlusselement 13 eingesteckt, so soll das Anschlusselement 13 abgeschlossen und abgedichtet sein.

Dies wird mit dem Ventilelement 18 erreicht. Das Ventilelement 18 weist einen zylindrischen Bereich 24 auf, von dem nach unten Federarme 25 abragen. Dabei ist der zylindrische Bereich 24 mit den Federarmen 25 vorteilhaft einteilig ausgebildet und die Federarme 25 ragen in einem Winkel von etwa 45° zur Vertikalen nach radial außen und unten von dem zylindrischen Bereich 24 ab. Die Federarme 25 bilden bei Vorhandensein von drei Federarmen etwa eine Anordnung gemäß einer Kantenanordnung eines Tetraeders.

Die abragenden Federarme 25 sind vorteilhaft als gerade oder einfach oder mehrfach abgeknickte oder gekrümmte Federarme 25 ausgebildet. Im Ausführungsbeispiel der Figuren 1 bis 6 sind die Federarme 25 zweifach abgeknickt ausgebildet.

Dazu weisen die Federarme 25 einen ersten Bereich 26 auf, welcher in einem Winkel a1 von etwa 25 bis 30°, vorzugsweise 27°, zur Ebene senkrecht zur Längsachse des zylindrischen Bereichs 24 angeordnet ist.

Weiterhin weisen die Federarme 25 einen zweiten Bereich 27 auf, welcher in einem Winkel a2 von etwa 35 bis 40°, vorzugsweise 37°, zur Ebene senkrecht zur Längsachse des zylindrischen Bereichs 24 angeordnet ist.

Auch weisen die Federarme einen dritten Bereich 28 auf, welcher in einem Winkel a3 von etwa 75 bis 80°, vorzugsweise 77°, zur Ebene senkrecht zur Längsachse des zylindrischen Bereichs 24 angeordnet ist.

Der zylindrische Bereich 24 weist einen Kanal 29 auf, welcher an seinem oberen Ende verschlossen ist. Dort weist der zylindrische Bereich 24 einen Schlitz 30 auf, welcher bei Verformung öffnet.

Ist der Schlitz 30 verschlossen, so ist eine Fluidverbindung zwischen dem Anschlusselement 13 und dem Aufnahmeraum 16 unterbunden. Ist der Schlitz 30 geöffnet, so ist eine Fluidverbindung zwischen dem Anschlusselement 13 und dem Aufnahmeraum 16 ermöglicht.

Wird das Ventilelement 18 durch ein zu verbindendes Element in axialer Richtung nach unten gedrückt, so greift der Hohldorn 20 in den Kanal 29 ein und verformt den zylindrischen Bereich 24, so dass der Schlitz geöffnet wird.

In der in Figur 1 gezeigten Positionen des Ventilelements 15 ist eine Fluidverbindung zwischen dem Anschlusselement 13 und dem Aufnahmeraum 16 abgeschlossen und abgedichtet. Wird von oben in das Anschlusselement 13 ein Element eingesteckt, so wird der zylindrische Bereich 24 des Ventilelements 18 komprimiert und nach unten geschoben, so dass sich die Federarme 25 an den Rändern der Taschen 17 abstützen und sich elastisch verformen. Dadurch wird der zylindrische Bereich 24 nach unten verlagert und die Fluidverbindung wird mittels Schlitz 30 und Kanal 29 und den Hohldorn ermöglicht.

Wird das zu verbindende Element wieder aus dem Anschlusselement 7 entfernt, so entspannt sich das elastische formbare Ventilelement 18 wieder und der zylindrische Bereich 24 wird wieder nach oben geschoben, so dass das Anschlusselement 13 wieder abgedichtet ist.

Der Innenraum des Verbindungselements 1 wird durch den Boden 19 geteilt. Unterhalb des Bodens 19 ist der Aufnahmeraum 23 angeordnet, welcher etwa zylindrisch ausgebildet ist. In diesem Aufnahmeraum 23 ist das zweite Ventilelement 31 mit dem zylindrischen Bereich 32 und den Federarmen 33 angeordnet. Die Federarme sind zwischen einer Flanke des ersten Gehäuseteils und dem Boden 19 angeordnet und stützen den zylindrischen Bereich 32 in axialer Richtung ab. Wird in den Anschlussbereich 8 ein zu verbindendes Element geschraubt, so wird der zylindrische Bereich 32 des zweiten Ventilelements entgegen der Rückstellkraft der Federarme 33 axial nach oben verlagert, wobei der zylindrische Bereich 32 auch verformt wird. Dabei löst sich der untere Dichtbereich 34 vom umlaufenden Wulst 35 des rohrförmigen Bereichs 11 und eine Fluidströmung wird zugelassen.

Bevorzugt sind die elastischen Elemente, die als Ventilelemente 18, 31 vorgesehen sind, aus einem elastomeren Material und die Gehäuseelemente 3, 4, 5 aus einem nicht elastomeren, eher formstabilen, Material, wie aus einem Thermoplast. Dabei kann das Element 18 und das Element 31 bevorzugt aus Liquid Silicone Rubber (LSR) oder Hochtemperatur vernetzendem Silikonkautschuk (HTV) oder Raumtemperatur vernetzendem Silikonkautschuk (RTV) bestehen und die Gehäuseteile 3, 4, 5 können aus Acrylnitril/Butadien/Styrol (ABS), Polycarbonat (PC), Polypropylen (PP) oder Polyethylen (PE) o.ä. bestehen.

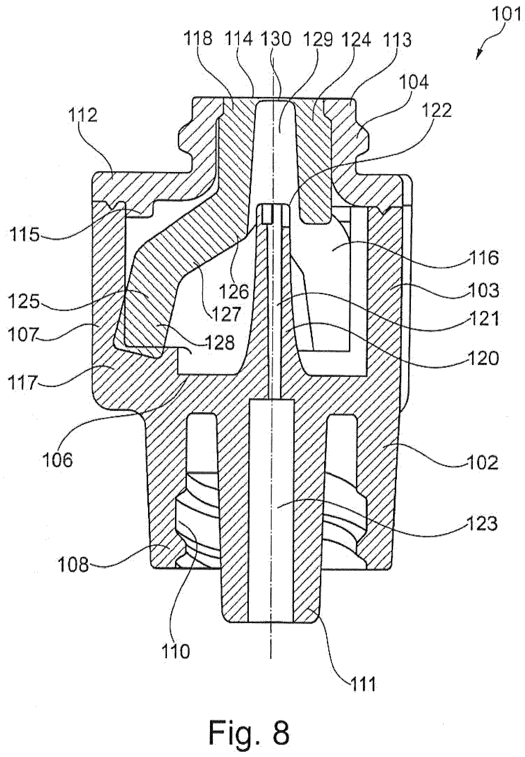

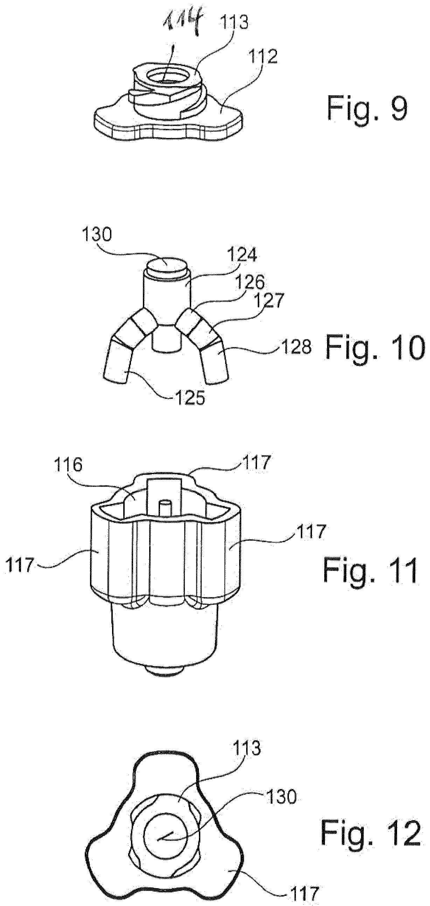

Die Figuren 8 bis 12 zeigen ein nicht erfindungsgemäßes Beispiel eines Verbindungselements 101 in verschiedenen Darstellungen. Die Figur 8 zeigt das Verbindungselement 101 im Schnitt, die Figuren 9 bis 11 zeigen eine Explosionsdarstellung in perspektivischer Ansicht, die Figur 12 zeigt eine Aufsicht.

Das Verbindungselement 101 ist mit einem Gehäuse 102 ausgebildet. Das Gehäuse 102 besteht aus zumindest zwei Gehäuseteilen 103, 104. Die Gehäuseteile 103, 104, sind miteinander verbunden, wie miteinander verschweißt ausgebildet. Statt eines zusätzlichen Gehäuseteils ist der Hohldorn in diesem Ausführungsbeispiel einteilig mit dem Gehäuseteil 103 ausgebildet. Das erste Gehäuseteil 103 weist einen Boden 106 mit einer daran im Winkel von 90° abragenden umlaufenden Wand 107 auf. Die Wand 107 ist dabei im Wesentlichen axial ausgerichtet.

Der Boden 105 ist mit dem Anschlusselement 108 versehen. Dazu ist das Anschlusselement 108 als Anschlussstutzen an dem Boden 106 angebracht. Vorteilhaft ist es mit dem Boden 106 einteilig verbunden oder ausgebildet. Dabei kann mit dem Anschlusselement 108 ein zu verbindendes Element verbunden, wie verschraubt, werden. Dazu ist ein Gewinde 110 vorgesehen, mittels welchem das zu verbindende Element verschraubt werden kann.

Das Gehäuseteil 103 weist einen rohrförmigen Bereich 111 auf, welcher in axialer Richtung von dem Boden 106 abragt und in entgegengesetzter Richtung der Wand 107 vom Boden 106 abragt.

Das zweite Gehäuseteil 104 ist deckelartig mit einem Boden 112 ausgebildet, von dem sich in axialer Richtung nach oben ein Anschlussstutzen 113 mit einer Fluidöffnung 114 erstreckt. In die entgegengesetzte Richtung ist ein Vorsprung 115 vorgesehen, der in die Wand 107 radial innen und in axialer Richtung eingreift. Der Vorsprung 115 ist, wie in Figur 9 dargestellt, segmentartig ausgebildet und über den Umfang des Bodens 112 verteilt.

Wie in der Figur 8 zu erkennen ist, sind die beiden Gehäuseteile 103, 104 an der Stirnseite der Wand 107 miteinander verbunden, wie verschweißt. Auch kann alternativ ein Verkleben, Verpressen, Verclipsen etc. anwendbar sein.

Das Gehäuse 102 bildet einen ersten Aufnahmeraum 116, wobei dieser Aufnahmeraum 116 innerhalb des Gehäuseteils 103 im vom Gehäuseteil 104 abgeschlossenen Bereich angeordnet ist. Dabei wird der Aufnahmeraum 116 durch die Wand 107 umgeben.

Die Wand 107 ist gemäß der Figuren 8 und 11 nicht vollständig rund ausgebildet, sondern weist nach radial außen vorstehende Taschen 117 auf, die zur Aufnahme von Federarmen des Ventilelements dienen. Dabei sind die Taschen 117 als nach radial außen abstehende Bereiche eines Kreises ausgebildet, wobei der Radius der Wand 107 zwischen den Taschen 117 geringer ist.

Im Aufnahmeraum 116 ist ein von dem Boden 106 abragender Hohldorn 120 aufgenommen, welcher einen durchgehenden Kanal 121 aufweist. Am oberen Ende des Hohldorns weist der Dorn zwei gegenüberliegende Vorsprünge 122 auf, die den Kanal zwischen sich in lateraler Richtung verlaufend enden lassen. Der Kanal 121 ist hin zum zweiten Aufnahmeraum 123 offen.

Im Aufnahmeraum 116 ist weiterhin ein Ventilelement 118 angeordnet.

Nach oben am Boden 112 des Gehäuseteils 104 ist das Anschlusselement 113 vorgesehen, wie bereits oben beschrieben. Wird ein mit dem Anschlusselement 113 zu verbindendes Element, wie beispielsweise eine Spritze oder ähnliches, bzw. ein daran angebrachter Stutzen in das Anschlusselement 113 eingesteckt oder eingeschraubt, so soll eine Fluidkommunikation zwischen dem zu verbindenden Element und dem Aufnahmeraum 116 erfolgen können. Wird kein zu verbindendes Element in das Anschlusselement 113 eingesteckt, so soll das Anschlusselement 113 abgeschlossen und abgedichtet sein.

Dies wird mit dem Ventilelement 118 erreicht. Das Ventilelement 118 weist einen zylindrischen Bereich 124 auf, von dem nach unten Federarme 125 abragen. Dabei ist der zylindrische Bereich 124 mit den Federarmen 125 vorteilhaft einteilig ausgebildet und die Federarme 125 ragen in einem Winkel von etwa 45° zur Vertikalen nach radial außen und unten von dem zylindrischen Bereich 124 ab. Die Federarme 125 bilden bei Vorhandensein von drei Federarmen etwa eine Anordnung gemäß einer Kantenanordnung eines Tetraeders.

Die abragenden Federarme 125 sind vorteilhaft als gerade oder einfach oder mehrfach abgeknickte oder gekrümmte Federarme 125 ausgebildet. Im Ausführungsbeispiel der Figuren 8 bis 12 sind die Federarme 125 zweifach abgeknickt ausgebildet.

Dazu weisen die Federarme 125 einen ersten Bereich 126 auf, welcher in einem Winkel α1 von etwa 25 bis 30°, vorzugsweise 27°, zur Ebene senkrecht zur Längsachse des zylindrischen Bereichs 124 angeordnet ist.

Weiterhin weisen die Federarme 125 einen zweiten Bereich 127 auf, welcher in einem Winkel a2 von etwa 35 bis 40°, vorzugsweise 37°, zur Ebene senkrecht zur Längsachse des zylindrischen Bereichs 124 angeordnet ist.

Auch weisen die Federarme einen dritten Bereich 128 auf, welcher in einem Winkel a3 von etwa 75 bis 80°, vorzugsweise 77°, zur Ebene senkrecht zur Längsachse des zylindrischen Bereichs 124 angeordnet ist.

Der zylindrische Bereich 124 weist einen Kanal 129 auf, welcher an seinem oberen Ende verschlossen ist. Dort weist der zylindrische Bereich 124 einen Schlitz 130 auf, welcher bei Verformung öffnet.

Ist der Schlitz 130 verschlossen, so ist eine Fluidverbindung zwischen dem Anschlusselement 113 und dem Aufnahmeraum 116 unterbunden. Ist der Schlitz 130 geöffnet, so ist eine Fluidverbindung zwischen dem Anschlusselement 113 und dem Aufnahmeraum 116 ermöglicht.

Wird das Ventilelement 118 durch ein zu verbindendes Element in axialer Richtung nach unten gedrückt, so greift der Hohldorn 120 in den Kanal 129 ein und verformt den zylindrischen Bereich 124, so dass der Schlitz geöffnet wird.

In der in Figur 8 gezeigten Position des Ventilelements 118 ist eine Fluidverbindung zwischen dem Anschlusselement 113 und dem Aufnahmeraum 116 abgeschlossen und abgedichtet. Wird von oben in das Anschlusselement 113 ein Element eingesteckt, so wird der zylindrische Bereich 124 des Ventilelements 118 komprimiert und nach unten geschoben, so dass sich die Federarme 125 an den Rändern der Taschen 117 abstützen und sich elastisch verformen. Dadurch wird der zylindrische Bereich 124 nach unten verlagert und die Fluidverbindung wird mittels Schlitz 130 und Kanal 129 und den Hohldorn ermöglicht.

Wird das zu verbindende Element wieder aus dem Anschlusselement 107 entfernt, so entspannt sich das elastische formbare Ventilelement 118 wieder und der zylindrische Bereich 124 wird wieder nach oben geschoben, so dass das Anschlusselement 113 wieder abgedichtet ist.

Der Innenraum des Verbindungselements 101 wird durch den Boden 106 geteilt. Unterhalb des Bodens 106 ist der Aufnahmeraum 123 angeordnet, welcher etwa zylindrisch ausgebildet ist. In diesem Aufnahmeraum 123 ist kein Ventilelement angeordnet. Wird in den Anschlussbereich 108 ein zu verbindendes Element geschraubt, so liegt eine Fluidverbindung zum Aufnahmeraum 116 vor.

Bevorzugt ist das elastische Element, das als Ventilelement 118 vorgesehen ist, aus einem elastomeren Material und die Gehäuseelemente 3, 4 aus einem nicht elastomeren, eher formstabilen, Material, wie aus einem Thermoplast. Dabei kann das Element 118 bevorzugt aus Liquid Silicone Rubber (LSR) oder Hochtemperatur vernetzendem Silikonkautschuk (HTV) oder Raumtemperatur vernetzendem Silikonkautschuk (RTV) bestehen und die Gehäuseteile 3, 4 können aus Acrylnitril/Butadien/Styrol (ABS), Polycarbonat (PC), Polypropylen (PP) oder Polyethylen (PE) o.ä. bestehen.

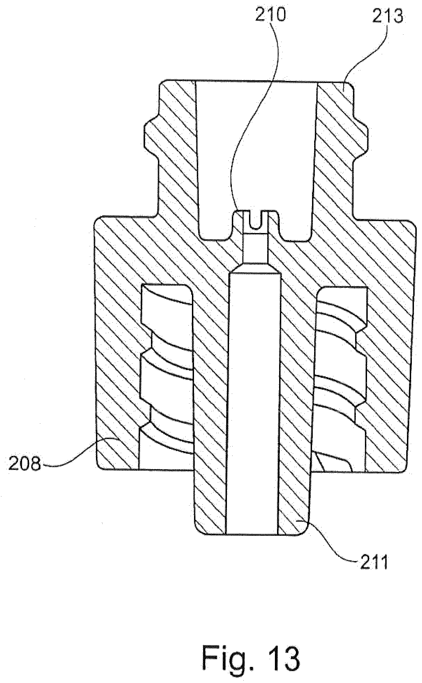

Die Figur 13 zeigt ein Verbindungselement 201, welches als Adapterteil dient. Es verbindet die Anschlusselemente 213 und 208 mit dem rohrförmigen Element 211, wie sie auch bereits aus den vorhergehenden Figuren bekannt sind, wobei in diesem Ausführungsbeispiel keine Ventilelemente vorgesehen sind. Wird das Anschlusselement 213 in das Anschlusselement 8 der Figur 1 verschraubt, so wird das zweite Ventilelement in axialer Richtung durch den Vorsprung 210 beaufschlagt und die Fluidverbindung wird durch Verlagerung und Verformung des zweiten Ventilelements geöffnet.

Vorstellbar ist eine Anordnung eines Verbindungselements 1 mit einem Verbindungselement 101 in einer Reihenschaltung. Dabei ist von oben auf den Anschlussbereich 13 ein zu verbindendes Element geschraubt, das jedoch nicht zu erkennen ist. Dadurch ist das zylindrische Element 24 nach unten verlagert und die Federarme 25 sind bogenförmig verspannt. Dabei ist auf das Verbindungselement 101 von oben das Verbindungselement 1 verschraubt, so dass der Anschlussbereich 8 auf den Anschlussbereich 113 geschraubt ist. Dadurch ist das zylindrische Element 124 nach unten verlagert und die Federarme 125 sind bogenförmig verspannt. Das zweite Verbindungselement 32 ist durch den Hohldorn 20 ebenso beaufschlagt und verformt und verlagert. Es besteht eine Fluidverbindung vom Anschlusselement 13 bis zum Anschlusselement 118.

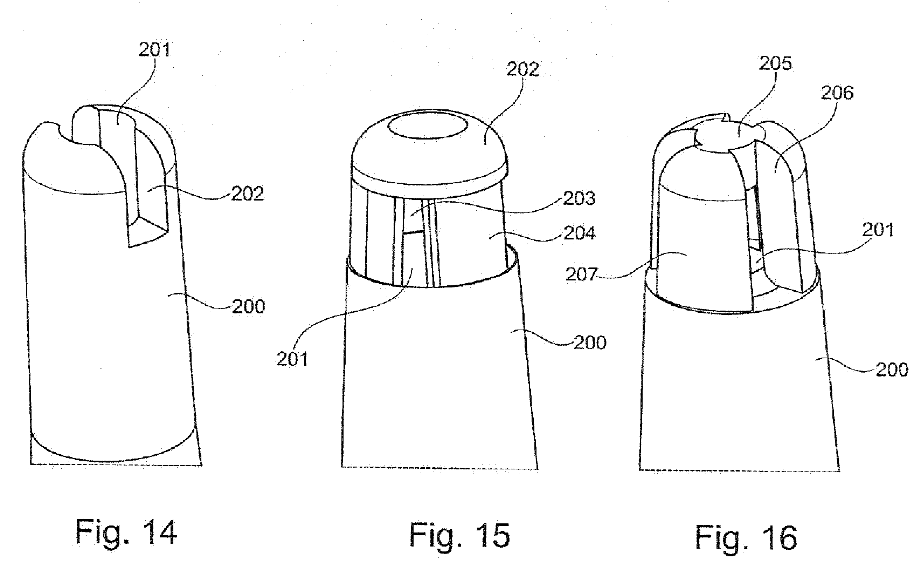

Die Figuren 14 bis 16 zeigen jeweils das obere Ende des Hohldorns 200, bei welchem die Öffnung jeweils unterschiedlich ausgebildet ist. In Figur 14 weist der Hohldorn 200 einen zylindrischen Kanal 201 auf, welcher durch eine quer zur Ausrichtung des Kanals vorgesehene Schlitzung 202 aufweist, so dass die Öffnung des Kanals nicht nur nach oben sondern auch zur Seite hin erfolgt.

In Figur 15 weist der Hohldorn 200 einen zylindrischen Kanal 201 auf, welcher an seinem Ende durch eine Kappe 202 abgedeckt ist, wobei seitlich und unterhalb der Kappe Öffnungen 203 ausgebildet sind zur Ausströmung des Fluids. Die Öffnungen 203 werden durch Stege 204 voneinander beabstandet.

In Figur 16 weist der Hohldorn 200 ebenso einen zylindrischen Kanal 201 auf, welcher an seinem Ende durch eine Kappe 205 abgedeckt ist, wobei seitlich und sich bis in die Kappe nach oben erstreckend Öffnungen 206 ausgebildet sind zur Ausströmung des Fluids. Die Öffnungen 206 werden durch Stege 207 voneinander beabstandet.

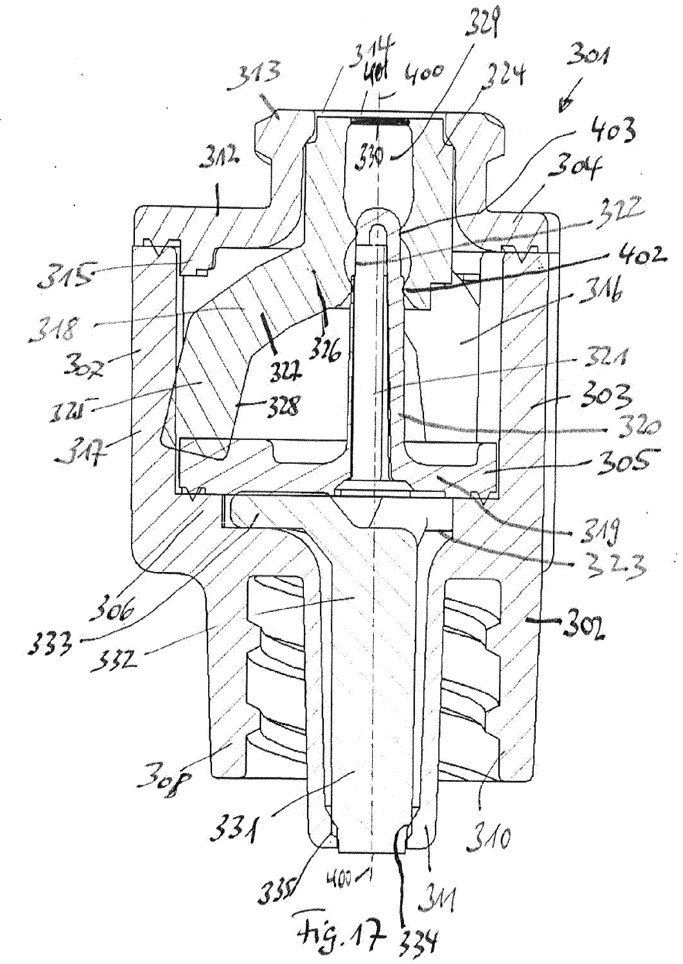

Die Figur 17 zeigt ein weiteres Ausführungsbeispiel eines erfindungsgemäßen Verbindungselements 301 im Schnitt.

Das Verbindungselement 301 ist mit einem Gehäuse 302 ausgebildet. Das Gehäuse 302 besteht aus zumindest zwei Gehäuseteilen, wobei in diesem Ausführungsbeispiel drei Gehäuseteile 303, 304, 305 vorgesehen sind. Die Gehäuseteile 303, 304, sind miteinander verbunden, wie miteinander verschweißt oder verklebt, ausgebildet. Das Gehäuseteil 305 ist in das Gehäuseteil 303 eingesetzt und auch dort mit dem Gehäuseteil 303 optional verbunden. Die Verbindung des Gehäuseteils 303 mit dem Gehäuseteil 305 kann alternativ auch entfallen, wie dies entsprechend auch mit den anderen Ausführungsbeispielen sein könnte. Als Verbindung könnte in allen Beispielen ein Verkleben, Verschweißen oder Klemmen oder ein formschlüssiges Verbinden möglich sein.

Das erste Gehäuseteil 303 weist einen Boden 306 mit einer daran im Winkel von 90° abragenden umlaufenden Wand 307 auf. Die Wand 307 ist dabei im Wesentlichen axial ausgerichtet. Dies bedeutet, dass sie in axialer Richtung zur Mittlachse 400, wie parallel dazu ausgerichtet ist.

Der Boden 305 ist mit dem Anschlusselement 308 versehen. Dazu ist das Anschlusselement 308 als Anschlussstutzen an dem Boden 306 angebracht. Vorteilhaft ist es mit dem Boden 306 einteilig verbunden oder ausgebildet. Dabei kann mit dem Anschlusselement 308 ein zu verbindendes Element verbunden, wie verschraubt, werden. Dazu ist ein Gewinde 310 vorgesehen, mittels welchem das zu verbindende Element verschraubt werden kann.

Das Gehäuseteil 303 weist einen rohrförmigen Bereich 311 auf, welcher in axialer Richtung von dem Boden 306 abragt und in entgegengesetzter Richtung zu der Wand 307 vom Boden 306 abragt.

Der rohrförmige Bereich 311 ist radial innerhalb des Anschlusselements 308 angeordnet und wird von diesem umgriffen. Auch ragt der rohrförmige Bereich 311 aus dem Anschlusselement 308 in axialer Richtung heraus.

Das zweite Gehäuseteil 304 ist deckelartig mit einem Boden 312 ausgebildet, von dem sich in axialer Richtung nach oben, also von dem eine Kammer bildenden Aufnahmeraum 316 weg, ein Anschlussstutzen 313 mit einer Fluidöffnung 314 erstreckt. In die entgegengesetzte Richtung ist ein Vorsprung 315 vorgesehen, der in die Wand 307 radial innen und in axialer Richtung eingreift. Der Vorsprung 315 ist vorteilhaft segmentartig ausgebildet und über den Umfang des Bodens 312 verteilt oder alternativ auch umlaufend ausgebildet.

Wie in der Figur 17 zu erkennen ist, sind die beiden Gehäuseteile 303, 304 an der Stirnseite der Wand 307 miteinander verbunden, wie verschweißt. Auch kann alternativ ein Verkleben, Verpressen, Verclipsen etc. anwendbar sein.

Das Gehäuse 302 bildet einen ersten Aufnahmeraum 316, wobei dieser Aufnahmeraum innerhalb des Gehäuseteils 303 im vom Gehäuseteil 304 abgeschlossenen Bereich angeordnet ist. Dabei wird der Aufnahmeraum 316 durch die Wand 307 umgeben.

Die Wand 307 ist gemäß der Figuren 17 und entsprechend der Figuren 6, 7, 11 und 12 nicht vollständig rund ausgebildet, sondern weist nach radial außen vorstehende Taschen 317 auf, die zur Aufnahme von Federarmen des Ventilelements dienen. Dabei sind die Taschen 317 als nach radial außen abstehende Bereiche eines Kreises ausgebildet, wobei der Radius der Wand 307 zwischen den Taschen 317 geringer ist.

Im Aufnahmeraum 316 ist das dritte Gehäuseteil 305 angeordnet. Dieses weist eine Bodenplatte 319 und einen davon abragenden Hohldorn 320 auf, welcher einen durchgehenden Kanal 321 aufweist. Am oberen Ende des Hohldorns weist der Hohldorn 320 eine Art Kappe auf, wobei seitlich eine Öffnung 322 vorgesehen ist, um Fluid ein- oder ausströmen zu lassen. Der Kanal 321 ist hin zum zweiten Aufnahmeraum 323 offen.

Im Aufnahmeraum 316 ist weiterhin ein Ventilelement 318 angeordnet.

Nach oben am Boden 312 des Gehäuseteils 304 ist das Anschlusselement 313 vorgesehen, wie bereits oben beschrieben. Wird ein mit dem Anschlusselement 313 zu verbindendes Element, wie beispielsweise eine Spritze oder ähnliches, bzw. ein daran angebrachter Stutzen in das Anschlusselement 313 eingesteckt oder eingeschraubt, so soll eine Fluidkommunikation zwischen dem zu verbindenden Element und dem Aufnahmeraum 316 erfolgen können. Wird kein zu verbindendes Element in das Anschlusselement 313 eingesteckt, so soll das Anschlusselement 13 abgeschlossen und abgedichtet sein.

Dies wird mit dem Ventilelement 318 erreicht. Das Ventilelement 318 weist einen zylindrischen Bereich 324 auf, von dem nach unten Federarme 325 abragen. Dabei ist der zylindrische Bereich 324 mit den Federarmen 325 vorteilhaft einteilig ausgebildet und die Federarme 325 ragen in einem Winkel von etwa 45° zur Vertikalen nach radial außen und unten von dem zylindrischen Bereich 324 ab. Die Federarme 325 bilden bei Vorhandensein von drei Federarmen 325 etwa eine Anordnung gemäß einer Kantenanordnung eines Tetraeders.

Die abragenden Federarme 325 sind vorteilhaft als gerade oder einfach oder mehrfach abgeknickte oder gekrümmte Federarme 325 ausgebildet. Im Ausführungsbeispiel der Figur 17 sind die Federarme 325 zweifach abgeknickt ausgebildet.

Dazu weisen die Federarme 325 einen ersten Bereich 326 auf, welcher in einem Winkel α1 von etwa 25 bis 30°, vorzugsweise 27°, zur Ebene senkrecht zur Längsachse 400 des zylindrischen Bereichs 324 angeordnet ist.

Weiterhin weisen die Federarme 325 einen zweiten Bereich 327 auf, welcher in einem Winkel a2 von etwa 35 bis 40°, vorzugsweise 37°, zur Ebene senkrecht zur Längsachse 400 des zylindrischen Bereichs 324 angeordnet ist.
Auch weisen die Federarme einen dritten Bereich 328 auf, welcher in einem Winkel a3 von etwa 75 bis 80°, vorzugsweise 77°, zur Ebene senkrecht zur Längsachse 400 des zylindrischen Bereichs 324 angeordnet ist.

Der zylindrische Bereich 324 weist einen Kanal 329 auf, welcher an seinem oberen Ende durch eine Wandung 401 verschlossen ist. Dort weist der zylindrische Bereich 324 einen Schlitz 330 auf, welcher bei Verformung der Wandung 401 öffnet.

Ist der Schlitz 330 verschlossen, so ist eine Fluidverbindung zwischen dem Anschlusselement 313 und dem Aufnahmeraum 316 unterbunden. Ist der Schlitz 330 geöffnet, so ist eine Fluidverbindung zwischen dem Anschlusselement 313 und dem Aufnahmeraum 316 ermöglicht.

Wird das Ventilelement 318 durch ein zu verbindendes Element in axialer Richtung nach unten gedrückt, so greift der Hohldorn 320 in den Kanal 329 ein und verformt den zylindrischen Bereich 324, so dass der Schlitz geöffnet wird.

Die Figur 17 zeigt, dass der Kanal 329 im Wesentlichen zylindrisch oder konisch ausgebildet ist und sich in axialer Richtung erstreckt. An seinem Innenumfang weist der Kanal 329 zwei nach radial innen vorstehende ringförmige Dichtlippen 402, 403 oder Dichtwülste auf. Diese Dichtlippen 402, 403 ragen nach radial innen und liegen an dem Hohldorn 320 an und dichten diesen ab. Dabei ist in Figur 17 zu erkennen, dass die Dichtlippe 403 oberhalb der Öffnung 322 angeordnet ist und die Dichtlippe 402 unterhalb der Öffnung angeordnet ist, so dass die beiden Dichtlippen 402, 403 beiderseits, in axialer Richtung betrachtet, der Öffnung 322 des Hohldorns angeordnet sind.

Alternativ zu den beiden Dichtlippen 402, 403 kann auch nur eine solche Dichtlippe vorgesehen sein, oder es können auch mehr als zwei solcher Dichtlippen vorgesehen sein.

In der in Figur 17 gezeigten Positionen des Ventilelements 315 ist eine Fluidverbindung zwischen dem Anschlusselement 313 und dem Aufnahmeraum 316 abgeschlossen und abgedichtet. Wird von oben in das Anschlusselement 313 ein Element eingesteckt, so wird der zylindrische Bereich 324 des Ventilelements 318 komprimiert und/oder nach unten geschoben, so dass sich die Federarme 325 an den Rändern der Taschen 317 abstützen und sich elastisch verformen. Dadurch wird der zylindrische Bereich 324 nach unten verlagert und die Fluidverbindung wird mittels Schlitz 330 und Kanal 329 und den Hohldorn ermöglicht.

Wird das zu verbindende Element wieder aus dem Anschlusselement 307 entfernt, so entspannt sich das elastische formbare Ventilelement 318 wieder und der zylindrische Bereich 324 wird wieder nach oben geschoben, so dass das Anschlusselement 313 wieder abgedichtet ist.

Der Innenraum des Verbindungselements 301 wird durch den Boden 319 geteilt. Unterhalb des Bodens 319 ist der Aufnahmeraum 323 angeordnet, welcher etwa zylindrisch ausgebildet ist. In diesem Aufnahmeraum 23 ist das zweite Ventilelement 331 mit dem zylindrischen Bereich 332 und den Federarmen 333 angeordnet. Die Federarme sind zwischen einer Flanke des ersten Gehäuseteils und dem Boden 319 angeordnet und stützen den zylindrischen Bereich 332 in axialer Richtung ab. Wird in den Anschlussbereich 308 ein zu verbindendes Element geschraubt, so wird der zylindrische Bereich 332 des zweiten Ventilelements entgegen der Rückstellkraft der Federarme 333 axial nach oben verlagert, wobei der zylindrische Bereich 332 auch verformt wird. Dabei löst sich der untere Dichtbereich 334 vom umlaufenden Wulst 335 des rohrförmigen Bereichs 311 und eine Fluidströmung wird zugelassen.

Bevorzugt sind die elastischen Elemente, die als Ventilelemente 318, 331 vorgesehen sind, aus einem elastomeren Material und die Gehäuseelemente 303, 304, 305 aus einem nicht elastomeren, eher formstabilen, Material, wie aus einem Thermoplast. Dabei kann das Element 318 und das Element 331 bevorzugt aus Liquid Silicone Rubber (LSR) oder Hochtemperatur vernetzendem Silikonkautschuk (HTV) oder Raumtemperatur vernetzendem Silikonkautschuk (RTV) bestehen und die Gehäuseteile 303, 304, 305 können aus Acrylnitril/Butadien/Styrol (ABS), Polycarbonat (PC), Polypropylen (PP) oder Polyethylen (PE) o.ä. bestehen.

## Patentansprüche

1. Verbindungselement (1, 301) mit einem Gehäuse (2, 302) mit einem ersten Aufnahmeraum (16, 316) mit einem ersten Anschlusselement (13, 313) und einem darin angeordneten ersten verlagerbaren Ventilelement (18, 318) und mit einem zweiten Aufnahmeraum (23, 323) mit einem zweiten Anschlusselement (8, 308) mit einem darin angeordneten zweiten verlagerbaren Ventilelement (31, 331), der erste und der zweite Aufnahmeraum (16, 316, 23, 323) sind miteinander fluidverbunden, wobei das erste Ventilelement (18, 318) elastisch verformbar ausgebildet und verlagerbar ist und das zweite Ventilelement (31, 331) elastisch verformbar ausgebildet und verlagerbar ist, wobei das erste Ventilelement (18, 318) zwischen zwei Positionen verlagerbar ist, wobei in der ersten Position eine Fluidverbindung zwischen dem ersten Aufnahmeraum (16, 316) und dem ersten Anschlusselement (13, 313) durch das erste Ventilelement (18, 318) unterbrochen ist und in der zweiten Position eine Fluidverbindung zwischen dem ersten Aufnahmeraum (16, 316) und dem ersten Anschlusselement (13, 313) durch das erste Ventilelement (18, 318) geöffnet ist und wobei das zweite Ventilelement (31, 331) zwischen zwei Positionen verlagerbar ist, wobei in der ersten Position eine Fluidverbindung zwischen dem zweiten Aufnahmeraum (23, 323) und dem zweiten Anschlusselement (8, 308) durch das zweite Ventilelement (31, 331) unterbrochen ist und in der zweiten Position eine Fluidverbindung zwischen dem zweiten Aufnahmeraum (23, 323) und dem zweiten Anschlusselement (8, 308) durch das zweite Ventilelement (31, 331) geöffnet ist, wobei das erste Ventilelement (18, 318) mit einem Kanal (29, 329) ausgebildet ist, welcher in der ersten Position des ersten Ventilelements (18, 318) verschlossen ist und in der zweiten Position des ersten Ventilelements (18, 318) geöffnet ist, wobei ein Hohldorn (20, 320) in dem ersten Aufnahmeraum (16, 316) angeordnet ist und in den Kanal (29, 329) eingreift, **dadurch gekennzeichnet, dass** das erste Ventilelement (18, 318) an dem zylindrischen Bereich (24, 324) abragende Federarme (25, 325) aufweist.

2. Verbindungselement (1, 301) nach Anspruch 1, **dadurch gekennzeichnet, dass** das Gehäuse (2, 302) zumindest zweiteilig ausgebildet ist, wobei die zumindest zwei Gehäuseteile (3, 4, 303, 304) vorteilhaft miteinander verbunden sind, wie insbesondere nicht zerstörungsfrei lösbar miteinander verbunden sind.

3. Verbindungselement (1, 301) nach Anspruch 2, **dadurch gekennzeichnet, dass** der erste Aufnahmeraum (16, 316) und der zweite Aufnahmeraum (23, 323) von einem ersten und einem zweiten Gehäuseteil (3, 4, 303, 304) gebildet werden, wobei das erste Gehäuseteil (3, 303) den ersten Aufnahmeraum (16, 316) und den zweiten Aufnahmeraum (23, 323) bilden, wobei das zweite Gehäuseteil (4, 304) den ersten Aufnahmeraum (16, 316) deckelartig begrenzt.

4. Verbindungselement (1, 301) nach einem der vorhergehenden Ansprüche, **dadurch gekennzeichnet, dass** der Hohldorn (20, 320) in das erste oder das zweite Gehäuseteil (3, 4, 303, 304) als drittes Gehäuseteil (5, 305) eingesetzt ist.

5. Verbindungselement (1, 301) nach einem der vorhergehenden Ansprüche, **dadurch gekennzeichnet, dass** das den Hohldorn (20, 320) bildende dritte Gehäuseteil (5, 305) mit dem ersten oder dem zweiten Gehäuseteil (3, 4, 303, 304) stoffschlüssig verbunden ist.

6. Verbindungselement (1, 301) nach einem der vorhergehenden Ansprüche, **dadurch gekennzeichnet, dass** der Hohldorn (20, 320) mit dem ersten oder dem zweiten Gehäuseteil (3, 4, 303, 304) einteilig ausgebildet, wie insbesondere gespritzt ist.

7. Verbindungselement (1, 301) nach einem der vorhergehenden Ansprüche, **dadurch gekennzeichnet, dass** das erste Anschlusselement (13, 313) an dem ersten Gehäuseteil (3, 303) angeordnet oder ausgebildet ist und das zweite Anschlusselement (8, 308) an dem zweiten Gehäuseteil (4, 304) angeordnet oder ausgebildet ist.

8. Verbindungselement (1, 301) nach zumindest einem der vorhergehenden Ansprüche, **dadurch gekennzeichnet, dass** der erste Aufnahmeraum (16, 316) einen im Schnitt kreisförmigen Querschnitt aufweist und von einer umlaufenden Wand begrenzt ist, wobei der Aufnahmeraum (16, 316) von zwei etwa ebenen Wandbereichen begrenzt wird, wobei das erste Anschlusselement (13, 313) an einer der beiden Wände angeordnet oder aufgenommen ist und in der anderen Wand eine Fluidverbindung zu dem zweiten Aufnahmeraum (23, 323) vorgesehen ist.

9. Verbindungselement (1, 301) nach zumindest einem der vorhergehenden Ansprüche, **dadurch gekennzeichnet, dass** der erste Aufnahmeraum (16, 316) einen im Schnitt kreisförmigen Querschnitt mit nach außen ragenden Taschen (17, 317) aufweist und von einer im Radius veränderlichen umlaufenden Wand begrenzt ist, wobei der Aufnahmeraum (16, 316) von zwei etwa ebenen Wandbereichen begrenzt wird, wobei das erste Anschlusselement (13, 313) an einer der beiden Wände angeordnet oder aufgenommen ist und in der anderen Wand eine Fluidverbindung zu dem zweiten Aufnahmeraum (23, 323) vorgesehen ist.

10. Verbindungselement (1, 301) nach zumindest einem der vorhergehenden Ansprüche, **dadurch gekennzeichnet, dass** die Anzahl der Taschen (17, 317) größer oder gleich der Anzahl von Federarmen (25, 325) eines in den Aufnahmeraum (16, 316) eingesetzten Ventilelements (18, 318) ist.

11. Verbindungselement (1, 301) nach Anspruch 10, **dadurch gekennzeichnet, dass** in jeder Tasche (17, 317) ein Federarm (25, 325) des Ventilelements (18, 318) angeordnet ist.

12. Verbindungselement (1, 301) nach einem der Ansprüche 1 bis 11, **dadurch gekennzeichnet, dass** die Federarme (25, 325) im Wesentlichen den Kanten eines Tetraeders entsprechend von dem zylindrischen Bereich (24, 324) abragen.

13. Verbindungselement (1, 301) nach einem der Ansprüche 1 bis 12, **dadurch gekennzeichnet, dass** die abragenden Federarme (25, 325) als gerade oder einfach oder mehrfach abgeknickte oder gekrümmte Federarme (25, 325) ausgebildet sind.

14. Verbindungselement (1, 301) nach einem der vorhergehenden Ansprüche, **dadurch gekennzeichnet, dass** die Federarme (25, 325) des ersten Ventilelements (18, 318) in einem Winkel von 120° zueinander in der Ebene senkrecht zur Längsachse (400) des zylindrischen Bereichs (24, 324) angeordnet sind.

15. Verbindungselement (1, 301) nach einem der vorhergehenden Ansprüche, **dadurch gekennzeichnet, dass** die Federarme (25, 325) einen ersten Bereich (26, 326) aufweisen, welcher in einem Winkel von etwa 25 bis 30°, vorzugsweise 27°, zur Ebene senkrecht zur Längsachse (400) des zylindrischen Bereichs (24, 324) angeordnet ist.

16. Verbindungselement (1, 301) nach einem der vorhergehenden Ansprüche, **dadurch gekennzeichnet, dass** die Federarme (25, 325) einen zweiten Bereich (27, 327) aufweisen, welcher in einem Winkel von etwa 35 bis 40°, vorzugsweise 37°, zur Ebene senkrecht zur Längsachse (400) des zylindrischen Bereichs (24, 324) angeordnet ist.

17. Verbindungselement (1, 301) nach einem der vorhergehenden Ansprüche, **dadurch gekennzeichnet, dass** die Federarme (25, 325) einen dritten Bereich (28, 328) aufweisen, welcher in einem Winkel von etwa 75 bis 80°, vorzugsweise 77°, zur Ebene senkrecht zur Längsachse (400) des zylindrischen Bereichs (24, 324) angeordnet ist.

18. Verbindungselement (1, 301) nach einem der vorhergehenden Ansprüche, **dadurch gekennzeichnet, dass** die Federarme (25, 325) bei Belastung auf den zylindrischen Bereich (24, 324) sich derart bogenförmig verformen, dass der zylindrische Bereich (24, 324) im Wesentlichen eine axiale Bewegung vollzieht.

19. Verbindungselement (1, 301) nach einem der vorhergehenden Ansprüche, **dadurch gekennzeichnet, dass** der Kanal (29, 329) des Ventilelements (18, 318) auf einer Seite offen ist und auf der gegenüberliegenden Seite mit einem verschließbaren und öffenbaren Schlitz (30, 330) verschließbar ausgebildet ist.

20. Verbindungselement (1, 301) nach zumindest einem der vorhergehenden Ansprüche, **dadurch gekennzeichnet, dass** der Hohldorn (20, 320) in den Kanal (29, 329) im unbelasteten Zustand des Ventilelements (18, 318) nicht oder nur teilweise eingreift und den Schlitz (30, 330) im geschlossenen Zustand nicht beaufschlagt, so dass der Kanal (29, 329) verschlossen ist.

21. Verbindungselement (1, 301) nach zumindest einem der vorhergehenden Ansprüche, **dadurch gekennzeichnet, dass** der Hohldorn (20, 320) in den Kanal (29, 329) im belasteten Zustand des Ventilelements (18, 318) nahezu vollständig ein- oder hindurchgreift und den Schlitz (30, 330) beaufschlagt oder durchstößt, so dass der Kanal (29, 329) geöffnet ist.

22. Verbindungselement (1, 301) nach zumindest einem der vorhergehenden Ansprüche, **dadurch gekennzeichnet, dass** das zweite Anschlusselement (8, 308) ein hohlzylindrisches Element aufweist.

23. Verbindungselement (1, 301) nach zumindest einem der vorhergehenden Ansprüche, **dadurch gekennzeichnet, dass** das zweite Anschlusselement (8, 308) ein hohlzylindrisches Element aufweist und das zweite Ventilelement (31, 331) zumindest einen zylindrischen Bereich (32, 332) aufweist, welcher in dem hohlzylindrischen Element verlagerbar und/oder verformbar aufgenommen ist.

24. Verbindungselement (1, 301) nach einem der vorhergehenden Ansprüche, **dadurch gekennzeichnet, dass** das zweite Ventilelement (31, 331) an dem zylindrischen Bereich (32, 332) abragende Federarme (33, 333) aufweist.

25. Verbindungselement (1, 301) nach Anspruch 24, **dadurch gekennzeichnet, dass** die Federarme (33, 333) im Wesentlichen den Kanten eines Tetraeders entsprechend von dem zylindrischen Bereich (32, 332) abragen.

26. Verbindungselement (1, 301) nach Anspruch 24 oder 25, **dadurch gekennzeichnet, dass** die abragenden Federarme (33, 333) als gerade oder einfach oder mehrfach abgeknickte oder gekrümmte Federarme (33, 333) ausgebildet sind.

27. Verbindungselement (1, 301) nach einem der vorhergehenden Ansprüche, **dadurch gekennzeichnet, dass** die Federarme (33, 333) des zweiten Ventilelements (31, 331) in einem Winkel von 120° zueinander in der Ebene senkrecht zur Längsachse (400) des zylindrischen Bereichs (32, 332) angeordnet sind.

28. Verbindungselement (1, 301) nach einem der vorhergehenden Ansprüche, **dadurch gekennzeichnet, dass** die Federarme (33, 333) einen ersten Bereich aufweisen, welcher in einem Winkel von etwa 35 bis 55° zur Ebene senkrecht zur Längsachse (400) des zylindrischen Bereichs (32, 332) geneigt angeordnet ist.

29. Verbindungselement (1, 301) nach einem der vorhergehenden Ansprüche, **dadurch gekennzeichnet, dass** die Federarme (33, 333) einen zweiten Bereich aufweisen, welcher in einer Ebene senkrecht zur Längsachse (400) des zylindrischen Bereichs (32, 332) angeordnet ist.

30. Verbindungselement (1, 301) nach einem der vorhergehenden Ansprüche, **dadurch gekennzeichnet, dass** die Federarme (33, 333) bei Belastung auf den zylindrischen Bereich (32, 332) sich derart bogenförmig verformen, dass der zylindrische Bereich (32, 332) im Wesentlichen eine axiale Bewegung vollzieht.

31. Verbindungselement (1, 301) nach einem der vorhergehenden Ansprüche, **dadurch gekennzeichnet, dass** sich der zylindrische Bereich (32, 332) des zweiten Ventilelements (31, 331) unter Belastung krümmt.

32. Verbindungselement (1, 301) nach einem der vorhergehenden Ansprüche, **dadurch gekennzeichnet, dass** der zweite Aufnahmeraum (23, 323) endseitig eine nach radial innen gerichtete Einschnürung (35, 335) aufweist, woran sich das zweite Ventilelement (31, 331) im unbelasteten Zustand dichtend anlegt.

33. Verbindungselement (1, 301) nach einem der vorhergehenden Ansprüche, **dadurch gekennzeichnet, dass** das erste Ventilelement (18, 318) an seinem Kanal (29, 329) zumindest eine nach radial innen abstehende Dichtlippe (402, 403), insbesondere zwei Dichtlippen (402, 403), aufweist, welche zur Abdichtung an dem Hohldorn (320) anlegbar ist bzw. sind.

34. Verbindungselement (1, 301) nach einem der vorhergehenden Ansprüche, **dadurch gekennzeichnet, dass** die Federarme (25, 325) des ersten Ventilelements (18, 318) in einem Winkel von 120° zueinander in der Ebene senkrecht zur Längsachse (400) des zylindrischen Bereichs (24, 324) angeordnet sind.

35. Verbindungselement (1, 301) nach einem der vorhergehenden Ansprüche, **dadurch gekennzeichnet, dass** die Federarme (25, 325) einen ersten Bereich (26, 326) aufweisen, welcher in einem Winkel von etwa 35 bis 55° zu einer Ebene senkrecht zur Längsachse (400) des zylindrischen Bereichs (24, 324) angeordnet ist.

36. Verbindungselement (1, 301) nach einem der vorhergehenden Ansprüche, **dadurch gekennzeichnet, dass** die Federarme (25, 325) einen zweiten Bereich (27, 327) aufweisen, welcher in einer Ebene senkrecht zur Längsachse (400) des zylindrischen Bereichs (24, 324) angeordnet ist.

37. Verbindungselement (1, 301) nach zumindest einem der vorhergehenden Ansprüche, **dadurch gekennzeichnet, dass** der Querschnitt eines Federarms (25, 325) rund oder oval oder mehreckig ausgebildet ist.

38. Verbindungselement (1, 301) nach Anspruch 37, **dadurch gekennzeichnet, dass** der Querschnitt des Federarms (25, 325) oval ist, wobei der Querschnitt in einer Ebene durch die Mittelsenkrechte des Federelements einen kleineren Durchmesser aufweist als in der dazu senkrechten Richtung.

39. Verbindungselement (1, 301) nach einem der vorhergehenden Ansprüche, **dadurch gekennzeichnet, dass** der zylindrische Körper (24, 324) an einem Endbereich, welcher gegenüberliegend der Federarme (25, 325) angeordnet ist, eine umlaufende Dichtlippe oder einen umlaufenden Dichtbereich aufweist.

40. Verbindungselement (1, 301) nach einem der vorhergehenden Ansprüche, **dadurch gekennzeichnet, dass** die Dichtlippe oder der Dichtbereich als umlaufender Absatz ausgebildet ist.

41. Verbindungselement (1, 301) nach einem der vorhergehenden Ansprüche, **dadurch gekennzeichnet, dass** in dem zylindrischen Körper (24, 324) ein Kanal (29, 329) vorgesehen ist, welcher zumindest eine nach radial innen abstehende Dichtlippe (402, 403), insbesondere zwei Dichtlippen (402, 403), aufweist, welche zur Abdichtung an dem Hohldorn (320) anlegbar ist bzw. sind.

## Claims

1. A connecting element (1, 301) with a housing (2, 302) having a first receiving space (16, 316) with a first joining element (13, 313) and a first shiftable valve element (18, 318) arranged therein, and having a second receiving space (23, 323) with a second joining element (8, 308) with a second shiftable valve element (31, 331) arranged therein, wherein the first and the second receiving space (16, 316, 23, 323) are fluidically connected to each other, wherein the first valve element (18, 318) is of elastically deformable design and is shiftable and the second valve element (31, 331) is of elastically deformable design and is shiftable, wherein the first valve element (18, 318) is shiftable between two positions, wherein, in the first position, a fluidic connection between the first receiving space (16, 316) and the first joining element (13, 313) is interrupted by the first valve element (18, 318) and, in the second position, a fluidic connection between the first receiving space (16, 316) and the first joining element (13, 313) is opened by the first valve element (18, 318) and wherein the second valve element (31, 331) is shiftable between two positions, wherein, in the first position, a fluidic connection between the second receiving space (23, 323) and the second joining element (8, 308) is interrupted by the second valve element (31, 331) and, in the second position, a fluidic connection between the second receiving space (23, 323) and the second joining element (8, 308) is opened by the second valve element (31, 331) wherein the first valve element (18, 318) is formed with a duct (29, 329) which is closed in the first position of the first valve element (18, 318) and is opened in the second position of the first valve element (18, 318), wherein a hollow pin (20, 320) is arranged in the first receiving space (16, 316) and engages in the duct (29, 329), **characterised in that** the first valve element (18, 318) has spring arms (25, 325) protruding from the cylindrical region (24, 324).

2. The connecting element (1, 301) according to claim 1, **characterised in that** the housing (2, 302) is of at least two-part design, wherein the at least two housing parts (3, 4, 303, 304) are advantageously connected to each other, for example, in particular, are connected to each other so as not to be releasable non-destructively.

3. The connecting element (1, 301) according to claim 2, **characterised in that** the first receiving space (16, 316) and the second receiving space (23, 323) are formed by a first and a second housing part (3, 4, 303, 304), wherein the first housing part (3, 303) forms the first receiving space (16, 316) and the second receiving space (23, 323), wherein the second housing part (4, 304) bounds the first receiving space (16, 316) in the manner of a cover.

4. The connecting element (1, 301) according to one of the preceding claims, **characterised in that** the hollow pin (20, 320) is inserted as a third housing part (5, 305) into the first or the second housing part (3, 4, 303, 304).

5. The connecting element (1, 301) according to one of the preceding claims, **characterised in that** the third housing part (5, 305) forming the hollow pin (20, 320) is connected in an integrally bonded manner to the first or the second housing part (3, 4, 303, 304) .

6. The connecting element (1, 301) according to one of the preceding claims, **characterised in that** the hollow pin (20, 320) is formed integrally with the first or the second housing part (3, 4, 303, 304), in particular is injection-moulded thereon.

7. The connecting element (1, 301) according to one of the preceding claims, **characterised in that** the first joining element (13, 313) is arranged or formed on the first housing part (3, 303) and the second joining element (8, 308) is arranged or formed on the second housing part (4, 304).

8. The connecting element (1, 301) according to at least one of the preceding claims, **characterised in that** the first receiving space (16, 316) has a circular cross-section in section and is bounded by an encircling wall, wherein the receiving space (16, 316) is bounded by two approximately flat wall regions, wherein the first joining element (13, 313) is arranged or received on one of the two walls and a fluidic connection to the second receiving space (23, 323) is provided in the other wall.

9. The connecting element (1, 301) according to at least one of the preceding claims, **characterised in that** the first receiving space (16, 316) has a circular cross-section in section with outwardly protruding pockets (17, 317) and is bounded by an encircling wall which is changeable in radius, wherein the receiving space (16, 316) is bounded by two approximately flat wall regions, wherein the first joining element (13, 313) is arranged or received on one of the two walls and a fluidic connection to the second receiving space (23, 323) is provided in the other wall.

10. The connecting element (1, 301) according to at least one of the preceding claims, **characterised in that** the number of pockets (17, 317) is greater than or equal to the number of spring arms (25, 325) of a valve element (18, 318) inserted into the receiving space (16, 316).

11. The connecting element (1, 301) according to claim 10, **characterised in that** a spring arm (25, 325) of the valve element (18, 318) is arranged in each pocket (17, 317).

12. The connecting element (1, 301) according to one of claims 1 to 11, **characterised in that** the spring arms (25, 325) protrude from the cylindrical region (24, 324) in a manner substantially corresponding to the edges of a tetrahedron.

13. The connecting element (1, 301) according to one of claims 1 to 12, **characterised in that** the protruding spring arms (25, 325) are designed as spring arms (25, 325) which are rectilinear or are kinked or curved once or multiple times.

14. The connecting element (1, 301) according to one of the preceding claims, **characterised in that** the spring arms (25, 325) of the first valve element (18, 318) are arranged at an angle of 120° to one another in the plane perpendicular to the longitudinal axis (400) of the cylindrical region (24, 324).

15. The connecting element (1, 301) according to one of the preceding claims, **characterised in that** the spring arms (25, 325) have a first region (26, 326) which is arranged at an angle of approximately 25 to 30°, preferably 27°, to the plane perpendicular to the longitudinal axis (400) of the cylindrical region (24, 324) .

16. The connecting element (1, 301) according to one of the preceding claims, **characterised in that** the spring arms (25, 325) have a second region (27, 327) which is arranged at an angle of approximately 35 to 40°, preferably 37°, to the plane perpendicular to the longitudinal axis (400) of the cylindrical region (24, 324) .

17. The connecting element (1, 301) according to one of the preceding claims, **characterised in that** the spring arms (25, 325) have a third region (28, 328) which is arranged at an angle of approximately 75 to 80°, preferably 77°, to the plane perpendicular to the longitudinal axis (400) of the cylindrical region (24, 324) .

18. The connecting element (1, 301) according to one of the preceding claims, **characterised in that,** in the event of a load being applied to the cylindrical region (24, 324), the spring arms (25, 325) are deformed in an arcuate manner such that the cylindrical region (24, 324) substantially carries out an axial movement.

19. The connecting element (1, 301) according to one of the preceding claims, **characterised in that** the duct (29, 329) of the valve element (18, 318) is open on one side and is designed to be closable on the opposite side with a closable and openable slot (30, 330) .

20. The connecting element (1, 301) according to at least one of the preceding claims, **characterised in that,** in the unloaded state of the valve element (18, 318), the hollow pin (20, 320) only partially engages, if at all, in the duct (29, 329) and does not act upon the slot (30, 330) in the closed state, and therefore the duct (29, 329) is closed.

21. The connecting element (1, 301) according to at least one of the preceding claims, **characterised in that,** in the loaded state of the valve element (18, 318), the hollow pin (20, 320) virtually completely engages in or reaches through the duct (29, 329) and acts upon or pushes through the slot (30, 330), and therefore the duct (29, 329) is opened.

22. The connecting element (1, 301) according to at least one of the preceding claims, **characterised in that** the second joining element (8, 308) has a hollow-cylindrical element.

23. The connecting element (1, 301) according to at least one of the preceding claims, **characterised in that** the second joining element (8, 308) has a hollow-cylindrical element and the second valve element (31, 331) has at least one cylindrical region (32, 332) which is accommodated in a shiftable and/or deformable manner in the hollow-cylindrical element.

24. The connecting element (1, 301) according to one of the preceding claims, **characterised in that** the second valve element (31, 331) has spring arms (33, 333) protruding at the cylindrical region (32, 332).

25. The connecting element (1, 301) according to claim 24, **characterised in that** the spring arms (33, 333) protrude from the cylindrical region (32, 332) in a manner substantially corresponding to the edges of a tetrahedron.

26. The connecting element (1, 301) according to claim 24 or 25, **characterised in that** the protruding spring arms (33, 333) are designed as spring arms (33, 333) which are rectilinear or are kinked or curved once or multiple times.

27. The connecting element (1, 301) according to one of the preceding claims, **characterised in that** the spring arms (33, 333) of the second valve element (31, 331) are arranged at an angle of 120° to one another in the plane perpendicular to the longitudinal axis (400) of the cylindrical region (32, 332).

28. The connecting element (1, 301) according to one of the preceding claims, **characterised in that** the spring arms (33, 333) have a first region which is arranged inclined at an angle of approximately 35 to 55° to the plane perpendicular to the longitudinal axis (400) of the cylindrical region (32, 332).

29. The connecting element (1, 301) according to one of the preceding claims, **characterised in that** the spring arms (33, 333) have a second region which is arranged in a plane perpendicular to the longitudinal axis (400) of the cylindrical region (32, 332).

30. The connecting element (1, 301) according to one of the preceding claims, **characterised in that,** in the event of a load being applied to the cylindrical region (32, 332), the spring arms (33, 333) are deformed in an arcuate manner such that the cylindrical region (32, 332) substantially carries out an axial movement.

31. The connecting element (1, 301) according to one of the preceding claims, **characterised in that** the cylindrical region (32, 332) of the second valve element (31, 331) bends under a load.

32. The connecting element (1, 301) according to one of the preceding claims, **characterised in that** the end side of the second receiving space (23, 323) has a radially inwardly directed constriction (35, 335) against which the second valve element (31, 331) is positioned in a sealing manner in the unloaded state.

33. The connecting element (1, 301) according to one of the preceding claims, **characterised in that** the first valve element (18, 318) has, on its duct (29, 329), at least one radially inwardly protruding sealing lip (402, 403), in particular two sealing lips (402, 403), which can be positioned on the hollow pin (320) for sealing purposes.

34. The connecting element (1, 301) according to one of the preceding claims, **characterised in that** the spring arms (25, 325) of the first valve element (18, 318) are arranged at an angle of 120° to one another in the plane perpendicular to the longitudinal axis (400) of the cylindrical region (24, 324).

35. The connecting element (1, 301) according to one of the preceding claims, **characterised in that** the spring arms (25, 325) have a first region (26, 326) which is arranged at an angle of approximately 35 to 55° to a plane perpendicular to the longitudinal axis (400) of the cylindrical region (24, 324).

36. The connecting element (1, 301) according to one of the preceding claims, **characterised in that** the spring arms (25, 325) have a second region (27, 327) which is arranged in a plane perpendicular to the longitudinal axis (400) of the cylindrical region (24, 324) .

37. The connecting element (1, 301) according to at least one of the preceding claims, **characterised in that** the cross-section of a spring arm (25, 325) is of round or oval or polygonal design.

38. The connecting element (1, 301) according to claim 37, **characterised in that** the cross-section of the spring arm (25, 325) is oval, wherein the cross-section has a smaller diameter in a plane through the mean perpendicular of the spring element than in the direction perpendicular thereto.

39. The connecting element (1, 301) according to one of the preceding claims, **characterised in that** the cylindrical body (24, 324) has an encircling sealing lip or an encircling sealing region at an end region which is arranged opposite the spring arms (25, 325).

40. The connecting element (1, 301) according to one of the preceding claims, **characterised in that** the sealing lip or the sealing region is designed as an encircling step.

41. The connecting element (1, 301) according to one of the preceding claims, **characterised in that** a duct (29, 329) is provided in the cylindrical body (24, 324), said duct having at least one radially inwardly protruding sealing lip (402, 403), in particular two sealing lips (402, 403), which can be positioned on the hollow pin (320) for sealing purposes.

## Revendications

1. Elément de liaison (1, 301) comprenant un boîtier (2, 302) comportant un premier espace de logement (16, 316) ayant un premier élément de raccordement (13, 313) et un premier élément mobile formant soupape (18, 318) disposé à l'intérieur dudit premier espace de logement, et comportant un second espace de logement (23, 323) ayant un second élément de raccordement (8, 308) et ayant un second élément mobile formant soupape (31, 331) disposé à l'intérieur dudit second espace de logement, le premier et le second espace de logement (16, 316, 23, 323) étant reliés l'un à l'autre par communication fluidique, où le premier élément formant soupape (18, 318) est configuré en étant déformable élastiquement et en étant déplaçable, et le second élément formant soupape (31, 331) est configuré en étant déformable élastiquement et en étant déplaçable, où le premier élément formant soupape (18, 318) peut être déplacé entre deux positions où, dans la première position, une communication fluidique entre le premier espace de logement (16, 316) et le premier élément de raccordement (13, 313) est interrompue par le premier élément formant soupape (18, 318) et, dans la seconde position, une communication fluidique entre le premier espace de logement (16, 316) et le premier élément de raccordement (13, 313) est ouverte par le premier élément formant soupape (18, 318), et où le second élément formant soupape (31, 331) peut être déplacé entre deux positions où, dans la première position, une communication fluidique entre le second espace de logement (23, 323) et le second élément de raccordement (8, 308) est interrompue par le second élément formant soupape (31, 331) et, dans la seconde position, une communication fluidique entre le second espace de logement (23, 323) et le second élément de raccordement (8, 308) est ouverte par le second élément formant soupape (31, 331), où le premier élément formant soupape (18, 318) est conçu en comportant un canal (29, 329) qui, dans la première position du premier élément formant soupape (18, 318), est fermé et qui, dans la seconde position du premier élément formant soupape (18, 318) est ouvert, où un mandrin creux (20, 320) est disposé dans le premier espace de logement (16, 316) et s'engage dans le canal (29, 329), **caractérisé en ce que** le premier élément formant soupape (18, 318) présente des bras à ressort (25, 325) faisant saillie par rapport à la zone cylindrique (24, 324).

2. Elément de liaison (1, 301) selon la revendication 1, **caractérisé en ce que** le boîtier (2, 302) est configuré au moins en deux parties, où les parties de boîtier (3, 4, 303, 304) au moins au nombre de deux sont, de façon avantageuse, assemblées l'une à l'autre, telles qu'elles sont assemblées l'une à l'autre, en particulier en n'étant pas détachables de façon non destructive.

3. Elément de liaison (1, 301) selon la revendication 2, **caractérisé en ce que** le premier espace de logement (16, 316) et le second espace de logement (23, 323) sont formés par une première et par une deuxième partie de boîtier (3, 4, 303, 304), où la première partie de boîtier (3, 303) forme le premier espace de logement (16, 316) et le second espace de logement (23, 323), où la deuxième partie de boîtier (4, 304) délimite, à la façon d'un couvercle, le premier espace de logement (16, 316).

4. Elément de liaison (1, 301) selon l'une quelconque des revendications précédentes, **caractérisé en ce que** le mandrin creux (20, 320) est introduit, comme troisième partie de boîtier (5, 305), dans la première ou la deuxième partie de boîtier (3, 4, 303, 304).

5. Elément de liaison (1, 301) selon l'une quelconque des revendications précédentes, **caractérisé en ce que** la troisième partie de boîtier (5, 305) formant le mandrin creux (20, 320) est assemblée, par continuité de matière, avec la première ou la deuxième partie de boîtier (3, 4, 303, 304).

6. Elément de liaison (1, 301) selon l'une quelconque des revendications précédentes, **caractérisé en ce que** le mandrin creux (20, 320), qui est en particulier moulé par injection, est configuré en formant une seule et même pièce avec la première ou la deuxième partie de boîtier (3, 4, 303, 304).

7. Elément de liaison (1, 301) selon l'une quelconque des revendications précédentes, **caractérisé en ce que** le premier élément de raccordement (13, 313) est disposé ou configuré sur la première partie de boîtier (3, 303), et le second élément de raccordement (8, 308) est disposé ou configuré sur la deuxième partie de boîtier (4, 304).

8. Elément de liaison (1, 301) selon au moins l'une quelconque des revendications précédentes, **caractérisé en ce que** le premier espace de logement (16, 316) présente, en coupe, une section transversale de forme circulaire et est délimité par une paroi périphérique, où l'espace de logement (16, 316) est délimité par deux zones de paroi à peu près planes, où le premier élément de raccordement (13, 313) est disposé ou logé sur l'une des deux parois et, dans l'autre paroi, il est prévu une communication fluidique avec le second espace de logement (23, 323).

9. Elément de liaison (1, 301) selon au moins l'une quelconque des revendications précédentes, **caractérisé en ce que** le premier espace de logement (16, 316) présente, en coupe, une section transversale de forme circulaire, en ayant des poches (17, 317) faisant saillie vers l'extérieur, et ledit premier espace de logement est délimité par une paroi périphérique dont le rayon est variable, où l'espace de logement (16, 316) est délimité par deux zones de paroi à peu près planes, où le premier élément de raccordement (13, 313) est disposé ou logé sur l'une des deux parois, et il est prévu, dans l'autre paroi, une communication fluidique avec le second espace de logement (23, 323).

10. Elément de liaison (1, 301) selon au moins l'une quelconque des revendications précédentes, **caractérisé en ce que** le nombre de poches (17, 317) est supérieur ou égal au nombre de bras à ressort (25, 325) d'un élément formant soupape (18, 318) introduit dans l'espace de logement (16, 316).

11. Elément de liaison (1, 301) selon la revendication 10, **caractérisé en ce qu'**un bras à ressort (25, 325) de l'élément formant soupape (18, 318) est disposé dans chaque poche (17, 317).

12. Elément de liaison (1, 301) selon l'une quelconque des revendications 1 à 11, **caractérisé en ce que** les bras à ressort (25, 325) sont en saillie par rapport à la zone cylindrique (24, 324) correspondant pratiquement aux arêtes d'un tétraèdre.

13. Elément de liaison (1, 301) selon l'une quelconque des revendications 1 à 12, **caractérisé en ce que** les bras à ressort (25, 325) faisant saillie sont configurés comme des bras à ressort (25, 325) rectilignes ou bien coudés ou courbés une seule fois ou plusieurs fois.

14. Elément de liaison (1, 301) selon l'une quelconque des revendications précédentes, **caractérisé en ce que** les bras à ressort (25, 325) du premier élément formant soupape (18, 318) sont disposés en formant un angle de 120° l'un par rapport à l'autre, dans le plan perpendiculaire à l'axe longitudinal (400) de la zone cylindrique (24, 324).

15. Elément de liaison (1, 301) selon l'une quelconque des revendications précédentes, **caractérisé en ce que** les bras à ressort (25, 325) présentent une première zone (26, 326) qui est disposée suivant un angle compris à peu près entre 25° et 30°, de préférence égal à 27°, par rapport au plan perpendiculaire à l'axe longitudinal (400) de la zone cylindrique (24, 324).

16. Elément de liaison (1, 301) selon l'une quelconque des revendications précédentes, **caractérisé en ce que** les bras à ressort (25, 325) présentent une deuxième zone (27, 327) qui est disposée suivant un angle compris à peu près entre 35° et 40°, de préférence égal à 37°, par rapport au plan perpendiculaire à l'axe longitudinal (400) de la zone cylindrique (24, 324).

17. Elément de liaison (1, 301) selon l'une quelconque des revendications précédentes, **caractérisé en ce que** les bras à ressort (25, 325) présentent une troisième zone (28, 328) qui est disposée suivant un angle compris à peu près entre 75° et 80°, de préférence égal à 77°, par rapport au plan perpendiculaire à l'axe longitudinal (400) de la zone cylindrique (24, 324).

18. Elément de liaison (1, 301) selon l'une quelconque des revendications précédentes, **caractérisé en ce que** les bras à ressort (25, 325), sous l'effet d'une charge se produisant sur la zone cylindrique (24, 324), se déforment de façon arquée, de manière telle que la zone cylindrique (24, 324) exécute pratiquement un mouvement axial.

19. Elément de liaison (1, 301) selon l'une quelconque des revendications précédentes, **caractérisé en ce que** le canal (29, 329) de l'élément formant soupape (18, 318) est ouvert sur un côté et, sur le côté opposé, est configuré en pouvant être fermé avec une fente (30, 330) qui peut être fermée et ouverte.

20. Elément de liaison (1, 301) selon au moins l'une quelconque des revendications précédentes, **caractérisé en ce que** le mandrin creux (20, 320), quand l'élément formant soupape (18, 318) est à l'état non chargé, ne s'engage pas ou ne s'engage que de façon partielle dans le canal (29, 329) et ne sollicite pas la fente (30, 330) quand celle-ci est fermée, de sorte que le canal (29, 329) est fermé.

21. Elément de liaison (1, 301) selon au moins l'une quelconque des revendications précédentes, **caractérisé en ce que** le mandrin creux (20, 320), quand l'élément formant soupape (18, 318) est à l'état chargé, s'engage presque complètement dans le canal (29, 329), ou bien le traverse, et sollicite la fente (30, 330) ou s'enfonce dans celle-ci, de sorte que le canal (29, 329) est ouvert.

22. Elément de liaison (1, 301) selon au moins l'une quelconque des revendications précédentes, **caractérisé en ce que** le second élément de raccordement (8, 308) présente un élément cylindrique creux.

23. Elément de liaison (1, 301) selon au moins l'une quelconque des revendications précédentes, **caractérisé en ce que** le second élément de raccordement (8, 308) présente un élément cylindrique creux, et le second élément formant soupape (31, 331) présente au moins une zone cylindrique (32, 332) qui est logée en pouvant être déplacée et / ou déformée dans l'élément cylindrique creux.

24. Elément de liaison (1, 301) selon l'une quelconque des revendications précédentes, **caractérisé en ce que** le second élément formant soupape (31, 331) présente des bras à ressort (33, 333) faisant saillie par rapport à la zone cylindrique (32, 332).

25. Elément de liaison (1, 301) selon la revendication 24, **caractérisé en ce que** les bras à ressort (33, 333) sont en saillie par rapport à la zone cylindrique (32, 332) correspondant pratiquement aux arêtes d'un tétraèdre.

26. Elément de liaison (1, 301) selon la revendication 24 ou 25, **caractérisé en ce que** les bras à ressort (33, 333) faisant saillie sont configurés comme des bras à ressort (33, 333) rectilignes ou bien coudés ou courbés une seule fois ou plusieurs fois.

27. Elément de liaison (1, 301) selon l'une quelconque des revendications précédentes, **caractérisé en ce que** les bras à ressort (33, 333) du second élément formant soupape (31, 331) sont disposés en formant un angle de 120° l'un par rapport à l'autre, dans le plan perpendiculaire à l'axe longitudinal (400) de la zone cylindrique (32, 332).

28. Elément de liaison (1, 301) selon l'une quelconque des revendications précédentes, **caractérisé en ce que** les bras à ressort (33, 333) présentent une première zone qui est disposée de façon inclinée suivant un angle compris à peu près entre 35° et 55°, par rapport au plan perpendiculaire à l'axe longitudinal (400) de la zone cylindrique (32, 332).

29. Elément de liaison (1, 301) selon l'une quelconque des revendications précédentes, **caractérisé en ce que** les bras à ressort (33, 333) présentent une deuxième zone qui est disposée dans un plan perpendiculaire à l'axe longitudinal (400) de la zone cylindrique (32, 332).

30. Elément de liaison (1, 301) selon l'une quelconque des revendications précédentes, **caractérisé en ce que** les bras à ressort (33, 333), sous l'effet d'une charge se produisant sur la zone cylindrique (32, 332), se déforment de façon arquée, de manière telle que la zone cylindrique (32, 332) exécute pratiquement un mouvement axial.

31. Elément de liaison (1, 301) selon l'une quelconque des revendications précédentes, **caractérisé en ce que** la zone cylindrique (32, 332) du second élément formant soupape (31, 331) se courbe sous l'effet d'une charge.

32. Elément de liaison (1, 301) selon l'une quelconque des revendications précédentes, **caractérisé en ce que** le second espace de logement (23, 323) présente, côté extrémité, un rétrécissement (35, 335) dirigé vers l'intérieur dans le sens radial, rétrécissement sur lequel vient en appui, de façon étanche, le second élément formant soupape (31, 331) quand celui-ci est à l'état non chargé.

33. Elément de liaison (1, 301) selon l'une quelconque des revendications précédentes, **caractérisé en ce que** le premier élément formant soupape (18, 318) présente, sur son canal (29, 329), au moins une lèvre d'étanchéité (402, 403), en particulier deux lèvres d'étanchéité (402, 403), faisant saillie vers l'intérieur dans le sens radial, lèvre(s) d'étanchéité qui, pour assurer l'étanchéité, peut ou peuvent être appliquée(s) sur le mandrin creux (320).

34. Elément de liaison (1, 301) selon l'une quelconque des revendications précédentes, **caractérisé en ce que** les bras à ressort (25, 325) du premier élément formant soupape (18, 318) sont disposés en formant un angle de 120° l'un par rapport à l'autre, dans le plan perpendiculaire à l'axe longitudinal (400) de la zone cylindrique (24, 324).

35. Elément de liaison (1, 301) selon l'une quelconque des revendications précédentes, **caractérisé en ce que** les bras à ressort (25, 325) présentent une première zone (26, 326) qui est disposée en formant un angle compris à peu près entre 35° et 55°, par rapport à un plan perpendiculaire à l'axe longitudinal (400) de la zone cylindrique (24, 324).

36. Elément de liaison (1, 301) selon l'une quelconque des revendications précédentes, **caractérisé en ce que** les bras à ressort (25, 325) présentent une deuxième zone (27, 327) qui est disposée dans un plan perpendiculaire à l'axe longitudinal (400) de la zone cylindrique (24, 324).

37. Elément de liaison (1, 301) selon au moins l'une quelconque des revendications précédentes, **caractérisé en ce que** la section transversale d'un bras à ressort (25, 325) est configurée en étant ronde ou ovale ou polygonale.

38. Elément de liaison (1, 301) selon la revendication 37, **caractérisé en ce que** la section transversale du bras à ressort (25, 325) est ovale, où la section transversale présente, dans un plan passant par la médiatrice de l'élément à ressort, un diamètre plus petit que celui s'étendant dans la direction perpendiculaire audit plan.

39. Elément de liaison (1, 301) selon l'une quelconque des revendications précédentes, **caractérisé en ce que** le corps cylindrique (24, 324), au niveau d'une zone d'extrémité qui est disposée à l'opposé des bras à ressort (25, 325), présente une lèvre d'étanchéité périphérique ou une zone d'étanchéité périphérique.

40. Elément de liaison (1, 301) selon l'une quelconque des revendications précédentes, **caractérisé en ce que** la lèvre d'étanchéité ou la zone d'étanchéité est configurée comme un épaulement périphérique.

41. Elément de liaison (1, 301) selon l'une quelconque des revendications précédentes, **caractérisé en ce qu'**il est prévu, dans le corps cylindrique (24, 324), un canal (29, 329) qui présente au moins une lèvre d'étanchéité (402, 403), en particulier deux lèvres d'étanchéité (402, 403), faisant saillie vers l'intérieur dans le sens radial, lèvre(s) d'étanchéité qui, pour assurer l'étanchéité, peut ou peuvent être appliquée(s) sur le mandrin creux (320).
